# EUROPEAN PATENT APPLICATION

(11) **EP 4 703 372 A1**
(43) Date of publication of application: **04.03.2026**
(21) Application number: 24796924.9
(22) Date of filing: 18.04.2024
(51) Int. Cl.: C07K 14/195, C07K 1/22, C07K 16/00, C12N 1/21, C12N 15/31, C12N 15/63, C12P 21/02

(54) **IMMUNOGLOBULIN-BINDING PROTEIN**

(30) Priority: 24.04.2023 JP 2023070555
(71) Applicant: Tosoh Corporation, Yamaguchi 746-8501 (JP)
(72) Inventor: HASEMI, Takatoshi, Ayase-shi, Kanagawa 252-1123 (JP); IWASE, Akihiro, Ayase-shi, Kanagawa 252-1123 (JP); AIDA, Kazuki, Ayase-shi, Kanagawa 252-1123 (JP); TANAKA, Toru, Ayase-shi, Kanagawa 252-1123 (JP)
(74) Representative: Novagraaf International SA
(86) International application number: PCT/JP2024/015488
(87) International publication number: WO 2024/225177

(57) **Abstract**

The present disclosure relates to a protein comprising an amino acid sequence of an immunoglobulin-binding domain of Protein L derived from a bacterium of the genus Finegoldia, provided that in the amino acid sequence, the protein has one or more amino acid substitutions selected from at least (1) to (5) below, the protein having immunoglobulin-binding activity:
(1) substitution of the amino acid residue corresponding to tyrosine at position 42 of SEQ ID NO: 1 with histidine;
(2) substitution of the amino acid residue corresponding to lysine at position 22 of SEQ ID NO: 1 with glutamic acid;
(3) substitution of the amino acid residue corresponding to lysine at position 38 of SEQ ID NO: 1 with proline or aspartic acid;
(4) substitution of the amino acid residue corresponding to tyrosine at position 42 of SEQ ID NO: 1 with tryptophan or phenylalanine; and
(5) substitution of the amino acid residue corresponding to asparagine at position 44 of SEQ ID NO: 1 with proline.

## Description

### Technical Field

The present disclosure relates to a protein that specifically binds to immunoglobulins. More specifically, the present disclosure relates to an immunoglobulin-binding protein with improved binding selectivity for immunoglobulins belonging to a particular light-chain subgroup.

### Background Art

Antibody drugs are pharmaceutical products that utilize antibodies (immunoglobulins), which are molecules responsible for immune functions in the body of living organisms. By virtue of the diversity of the variable regions of antibodies, antibody drugs bind to their target molecules with high specificity and affinity. Antibody drugs are thus less likely to cause adverse reactions, and the range of their indications has also been expanding in recent years. As a result, the market for them is rapidly growing.

An example of a new form of antibodies used as antibody drugs is bispecific antibodies, which are capable of binding to two different antigens simultaneously. For example, a bispecific antibody that recognizes a cancer cell antigen and an effector cell antigen is expected to enhance antitumor effects by effectively binding these cells together. Examples of forms of bispecific antibodies include trifunctional antibodies, which are composed of an Fc region and two Fab regions each capable of binding to a different antigen, F(ab')₂ antibodies, in which the two Fab regions have been joined together by linker peptides or other means, single-chain Fv (scFv) antibodies, in which only the variable regions of the two Fab regions have been joined together by linker peptides or other means, and bispecific T cell-engaging (BiTE) antibodies (Non-Paten Literature 1).

The manufacture of an antibody drug includes a culture step and a purification step. In the culture step, modification of antibody-producing cells and optimization of culture conditions are attempted to improve productivity. In the purification step, furthermore, affinity chromatography, which specifically recognizes antibody molecules, is employed as partial purification, and subsequent intermediate purification, final purification and virus removal are performed to formulate the antibody into its dosage form.

The ligand protein used as the support for affinity chromatography is in many cases Protein A derived from a bacterium of the genus Staphylococcus and having the property of binding to antibodies (immunoglobulins). Protein A, however, is a protein that binds specifically to the Fc region of antibodies. Accordingly, it is not applicable to the purification of F(ab')₂, scFv, BiTE and other antibodies lacking an Fc region. By contrast, Protein L derived from a bacterium of the genus Finegoldia is a protein that binds to κ light chains of immunoglobulins. By using Protein L as the ligand protein, therefore, antibodies lacking an Fc region, which cannot be purified using Protein A as described above, can be purified.

When antibodies are formed from two types of light chains and two types of heavy chains, the total number of possible combinations of a light chain and a heavy chain is ten. When manufacturing a bispecific antibody, therefore, it is necessary to select a bispecific antibody composed of the correct one of these combinations (Non-Patent Literature 1). Examples of methods for preventing incorrect combinations include the Knob-into-Holes method (Non-Patent Literature 2), the Cross-mab method (Non-Patent Literature 3) and the use of a common light chain (Non-Patent Literature 3). All of these methods, however, require modification of the antibody molecules and thus have the problems of reduced flexibility in molecular design and lower expression and performance resulting from the modification.

An example of a method for selecting the correct combination of light chains without modifying the antibody molecules is the method of using a κ chain as one of the two light chains and a λ chain as the other and selectively isolating a heterogenous assembly by utilizing the difference in affinity for Protein L. In general, however, λ chains are inferior in stability and solubility compared with κ chains, κ chains belonging to subgroup 1 and subgroup 3 in particular (Non-Patent Literature 5). The use of a λ chain, therefore, may impair the stability of the bispecific antibody.

As stated above, κ chains belonging to subgroup 1 and subgroup 3 are superior in stability and solubility. A bispecific antibody in which one light chain is a κ chain belonging to subgroup 1 and the other light chain is a κ chain belonging to subgroup 3, therefore, is expected to achieve improved stability. So far, however, there is no known Protein L capable of binding selectively to immunoglobulins that include a κ light chain belonging to a particular subgroup; it has been difficult to select a bispecific antibody as described above using Protein L.

### Prior Art Literatures

### Non-Patent Literature

Non-Patent Literature 1: Ulrich Brinkmann et al., MAbs., 2017; 9: 182-212.
Non-Patent Literature 2: J B Ridgway et al., Protein Eng., 1996; 9: 617-621.
Non-Patent Literature 3: Christian Klein et al., MAbs., 2016; 8: 1010-1020.
Non-Patent Literature 4: A. margaret Merchant et al., Nature Biotechnology, 1998; 16: 677-681
Non-Patent Literature 5: Dae Young Kim et al., MAbs., 2014; 6: 219-235.

### Summary of the Invention

### Technical Problem

An object of the present disclosure is to provide an immunoglobulin-binding protein with improved binding selectivity for immunoglobulins having a light chain belonging to a particular subgroup, and an adsorbent on which the protein is immobilized.

### Solution to Problem

As a result of intensive studies to solve the above problem, the inventors identified amino acid residues in the immunoglobulin-binding domains of Protein L derived from a bacterium of the genus Finegoldia (FpL) that are responsible for light-chain subgroup selection, and discovered that substituting these amino acid residues with other particular amino acid residues improves binding selectivity for κ light chain Subgroup 1.

More specifically, the present disclosure includes, for example, the aspects described in [1] to [14] below.

[1] A protein comprising an amino acid sequence of an immunoglobulin-binding domain of Protein L derived from a bacterium of the genus Finegoldia, provided that in the amino acid sequence, the protein has one or more amino acid substitutions selected from at least (1) to (5) below, the protein having immunoglobulin-binding activity:
   (1) substitution of an amino acid residue corresponding to tyrosine at position 42 of SEQ ID NO: 1 with histidine;
   (2) substitution of an amino acid residue corresponding to lysine at position 22 of SEQ ID NO: 1 with glutamic acid;
   (3) substitution of an amino acid residue corresponding to lysine at position 38 of SEQ ID NO: 1 with proline or aspartic acid;
   (4) substitution of an amino acid residue corresponding to tyrosine at position 42 of SEQ ID NO: 1 with tryptophan or phenylalanine; and
   (5) substitution of an amino acid residue corresponding to asparagine at position 44 of SEQ ID NO: 1 with proline.
[2] The protein according to [1] above, wherein the protein is a protein as described in any of (a) to (c) below:
   (a) a protein including an amino acid sequence set forth in SEQ ID NO: 1, provided that in the amino acid sequence, the protein has one or more amino acid substitutions selected from at least (1) to (5) below, the protein having immunoglobulin-binding activity:
      (1) substitution of an amino acid residue corresponding to tyrosine at position 42 of SEQ ID NO: 1 with histidine;
      (2) substitution of an amino acid residue corresponding to lysine at position 22 of SEQ ID NO: 1 with glutamic acid;
      (3) substitution of an amino acid residue corresponding to lysine at position 38 of SEQ ID NO: 1 with proline or aspartic acid;
      (4) substitution of an amino acid residue corresponding to tyrosine at position 42 of SEQ ID NO: 1 with tryptophan or phenylalanine; and
      (5) substitution of an amino acid residue corresponding to asparagine at position 44 of SEQ ID NO: 1 with proline;
   (b) a protein including an amino acid sequence set forth in SEQ ID NO: 1, provided that in the amino acid sequence, the protein has one or more amino acid substitutions selected from at least the (1) to (5) and also includes one or several substitutions, deletions, insertions and/or additions of amino acid residues at one or several positions in addition to the (1) to (5), the protein having immunoglobulin-binding activity; and
   (c) a protein including an amino acid sequence having a homology of 70% or more to an amino acid sequence set forth in SEQ ID NO: 1 or a partial sequence thereof, provided that in the amino acid sequence, the protein has one or more amino acid substitutions selected from at least the (1) to (5), the protein having immunoglobulin-binding activity.
[3] The protein according to [1] or [2] above, further comprising at least one or more amino acid substitutions selected from (6) to (11) below:
   (6) substitution of an amino acid residue corresponding to asparagine at position 50 of SEQ ID NO: 1 with tyrosine;
   (7) substitution of an amino acid residue corresponding to glutamic acid at position 4 of SEQ ID NO: 1 with glycine;
   (8) substitution of an amino acid residue corresponding to lysine at position 7 of SEQ ID NO: 1 with alanine;
   (9) substitution of an amino acid residue corresponding to glutamic acid at position 52 of SEQ ID NO: 1 with leucine;
   (10) substitution of an amino acid residue corresponding to tyrosine at position 53 of SEQ ID NO: 1 with phenylalanine; and
   (11) substitution of an amino acid residue corresponding to alanine at position 69 of SEQ ID NO: 1 with valine.
[4] The protein according to [1] or [2] above, further comprising at least an amino acid substitution of (6) below and one or more amino acid substitutions selected from (7) to (11) below:
   (6) substitution of an amino acid residue corresponding to asparagine at position 50 of SEQ ID NO: 1 with tyrosine;
   (7) substitution of an amino acid residue corresponding to glutamic acid at position 4 of SEQ ID NO: 1 with glycine;
   (8) substitution of an amino acid residue corresponding to lysine at position 7 of SEQ ID NO: 1 with alanine;
   (9) substitution of an amino acid residue corresponding to glutamic acid at position 52 of SEQ ID NO: 1 with leucine;
   (10) substitution of an amino acid residue corresponding to tyrosine at position 53 of SEQ ID NO: 1 with phenylalanine; and
   (11) substitution of an amino acid residue corresponding to alanine at position 69 of SEQ ID NO: 1 with valine.
[5] The protein according to any of [1] to [4] above, further comprising at least an amino acid substitution of (12) below:
   (12) substitution of an amino acid residue corresponding to lysine at position 29 of SEQ ID NO: 1 with isoleucine.
[6] The protein according to [3] or [4] above, wherein the protein is a protein as described in any of (d) to (f) below:
   (d) a protein including an amino acid sequence set forth in SEQ ID NO: 70, provided that in the amino acid sequence, the protein has one or more amino acid substitutions selected from at least (1) to (5) below, the protein having immunoglobulin-binding activity:
      (1) substitution of an amino acid residue corresponding to tyrosine at position 42 of SEQ ID NO: 1 with histidine;
      (2) substitution of an amino acid residue corresponding to lysine at position 22 of SEQ ID NO: 1 with glutamic acid;
      (3) substitution of an amino acid residue corresponding to lysine at position 38 of SEQ ID NO: 1 with proline or aspartic acid;
      (4) substitution of an amino acid residue corresponding to tyrosine at position 42 of SEQ ID NO: 1 with tryptophan or phenylalanine; and
      (5) substitution of an amino acid residue corresponding to asparagine at position 44 of SEQ ID NO: 1 with proline;
   (e) a protein including an amino acid sequence set forth in SEQ ID NO: 70, provided that in the amino acid sequence, the protein has one or more amino acid substitutions selected from at least the (1) to (5) and also includes one or several substitutions, deletions, insertions and/or additions of amino acid residues at one or several positions in addition to the (1) to (5), the protein having immunoglobulin-binding activity; and
   (f) a protein including an amino acid sequence having a homology of 70% or more to an amino acid sequence set forth in SEQ ID NO: 70 or a partial sequence thereof, provided that in the amino acid sequence, the protein has one or more amino acid substitutions selected from at least the (1) to (5), the protein having immunoglobulin-binding activity.
[7] A polynucleotide encoding the protein according to any of [1] to [6] above.
[8] An expression vector comprising the polynucleotide according to [7] above.
[9] A recombinant host comprising (A) or (B) below:
   (A) a polynucleotide encoding the protein according to any of [1] to [6] above; or
   (B) an expression vector including a polynucleotide encoding the protein according to any of [1] to [6] above.
[10] The recombinant host according to [9] above, wherein the host is Escherichia coli.
[11] A method for manufacturing a protein having immunoglobulin-binding activity, the method comprising a step of culturing a recombinant host including a polynucleotide encoding the protein according to any of [1] to [6] above or a recombinant host including an expression vector including the polynucleotide to allow the protein to be expressed and a step of recovering the expressed protein.
[12] The manufacturing method according to [11] above, wherein the host is Escherichia coli.
[13] An immunoglobulin adsorbent comprising an insoluble support and the protein according to any of [1] to [6] above immobilized on the insoluble support.
[14] A method for separating an immunoglobulin contained in a solution, the method comprising a step of applying a solution containing the immunoglobulin to a column packed with the adsorbent according to [13] above to allow the immunoglobulin to be adsorbed onto the adsorbent and a step of eluting the immunoglobulin adsorbed on the adsorbent. Description of Embodiments

In the following, the present disclosure will be described in detail.

A protein according to the present disclosure is a particular type of immunoglobulin-binding protein. As used herein, "immunoglobulin-binding protein" refers to a protein having affinity for immunoglobulins. The protein according to the present disclosure, therefore, has affinity for particular types of immunoglobulins. Specifically, the protein according to the present disclosure may be one having affinity for κ light chains of immunoglobulins. That is, the protein according to the present disclosure may specifically have affinity for κ light chains of immunoglobulins. More specifically, the protein according to the present disclosure may be one having affinity for Vκ1 of immunoglobulins. That is, the protein according to the present disclosure may more specifically have affinity for immunoglobulins having Vκ1. "Vκ1" refers to κ light chain subgroup 1; in other words, it refers to a light chain belonging to subgroup 1. Herein, affinity for immunoglobulins is also referred to as "immunoglobulin-binding activity" or "antibody-binding activity." Immunoglobulin-binding activity can be measured by, for example, ELISA (Enzyme-Linked ImmunoSorbent Assay). ELISA can be conducted under, for example, the conditions described in the Examples section.

The protein according to the present disclosure is a protein comprising an amino acid sequence of an immunoglobulin-binding domain of Protein L derived from a bacterium of the genus Finegoldia (also referred to as "FpL"), provided that in the amino acid sequence, the protein has at least one amino acid substitution at a particular position. Hereinafter, an amino acid sequence of an immunoglobulin-binding domain of FpL having no amino acid substitution at a particular position is also referred to as "unmodified amino acid sequence," and an amino acid sequence of an immunoglobulin-binding domain of FpL having at least one amino acid substitution at a particular position is also referred to as "modified amino acid sequence." A modified amino acid sequence, therefore, may be an amino acid sequence that is identical to an unmodified amino acid sequence except that it has at least one amino acid substitution at a particular position. According to an embodiment of the present disclosure, furthermore, the protein may be, for example, a protein including an amino acid sequence that is identical to an unmodified amino acid sequence except that it has at least one amino acid substitution at a particular position. Moreover, according to an embodiment of the present disclosure, the protein may be, for example, a protein including a modified amino acid sequence. An unmodified amino acid sequence may be a naturally occurring amino acid sequence or may not. An unmodified amino acid sequence may be modified, for example to have desired characteristics. An unmodified amino acid sequence may have, for example, an amino acid substitution other than amino acid substitutions at particular positions.

An example of a bacterium of the genus Finegoldia from which FpL is derived is Finegoldia magna. Examples of immunoglobulin-binding domains of Protein L derived from Finegoldia magna include:
the B1 domain (the amino acid residues at positions 104 to 173 of GenBank No. AAA25612);
the B2 domain (the amino acid residues at positions 176 to 245 of GenBank No. AAA25612);
the B3 domain (the amino acid residues at positions 248 to 317 of GenBank No. AAA25612);
the B4 domain (the amino acid residues at positions 320 to 389 of GenBank No. AAA25612);
the B5 domain (the amino acid residues at positions 393 to 462 of GenBank No. AAA25612);
the C1 domain (the amino acid residues at positions 249 to 317 of SEQ ID NO: 106 (GenBank No. AAA67503);
the C2 domain (the amino acid residues at positions 320 to 389 of SEQ ID NO: 106);
the C3 domain (the amino acid residues at positions 394 to 463 of SEQ ID NO: 106; SEQ ID NO: 1); and
the C4 domain (the amino acid residues at positions 468 to 537 of SEQ ID NO: 106).

Herein, an unmodified amino acid sequence may be:
(i) the full-length amino acid sequence of an immunoglobulin-binding domain of FpL as described above; or may be
(ii) a partial amino acid sequence of the domain, as long as the sequence has binding activity to immunoglobulins.

It should be noted that regarding (ii) above, an immunoglobulin-binding domain of FpL is composed of four β-sheets, one α-helix, loop portions connecting them and an N-terminal loop region. However, amino acid residues in regions unrelated to binding to immunoglobulins, such as the N-terminal loop region, may be absent. As a specific example, it is known that the C3 domain of FpL (SEQ ID NO: 1) retains immunoglobulin-binding potential even after deletion of the amino acid residues from glutamic acid at position 1 to glutamic acid at position 9, which correspond to the N-terminal loop region (Housden N.G. et al. Biochemical Society Transaction, 2003; 31: 716-718). The partial amino acid sequence in (ii) above, therefore, only needs to include at least the amino acid sequence of the binding site for immunoglobulins. That is, an example of a partial amino acid sequence in (ii) above is a partial sequence including the amino acid sequence of the binding site for immunoglobulins.

A specific example of a modified amino acid sequence as defined herein is an amino acid sequence of an immunoglobulin-binding domain as described above, such as the amino acid sequence set forth in SEQ ID NO: 1, having at least one amino acid substitution at a particular position. According to an embodiment of the present disclosure, therefore, a specific example of a protein is a protein including the amino acid sequence of an immunoglobulin-binding domain as described above, such as the amino acid sequence set forth in SEQ ID NO: 1, provided that in the amino acid sequence, the protein has at least one amino acid substitution at a particular position. In other words, according to an embodiment of the present disclosure, the protein may be, for example, a protein including an amino acid sequence that is identical to the amino acid sequence of an immunoglobulin-binding domain as described above, such as the amino acid sequence set forth in SEQ ID NO: 1, except that it has at least one amino acid substitution at a particular position.

Herein, when a protein includes an amino acid sequence, this is also described as "a protein includes amino acid residues consisting of an amino acid sequence." When a protein or amino acid sequence has an amino acid substitution, furthermore, this is also described as "an amino acid substitution has occurred in a protein or amino acid sequence." Moreover, an amino acid that constitutes a protein or amino acid sequence are also referred to as "amino acid residue."

According to an embodiment of the present disclosure, the protein may be an immunoglobulin-binding protein with improved binding selectivity for Vκ1. According to an embodiment of the present disclosure, the protein may specifically be an immunoglobulin-binding protein that exhibits improved binding selectivity for Vκ1 compared with immunoglobulin-binding proteins having no modified amino acid sequence (e.g., consisting of an unmodified amino acid sequence). According to an embodiment of the present disclosure, the protein may specifically be an immunoglobulin-binding protein that exhibits improved binding selectivity for Vκ1 compared with immunoglobulin-binding proteins lacking the amino acid substitution at a particular position. In other words, according to an embodiment of the present disclosure, the protein may be an immunoglobulin-binding protein that exhibits improved binding selectivity for Vκ1 by virtue of having the amino acid substitution at a particular position. In other words, furthermore, the amino acid substitution at a particular position may be a substitution or substitutions that improve the binding selectivity for Vκ1 of the immunoglobulin-binding protein.

"Improved binding selectivity for Vκ1" may mean that affinity for Vκ1 relatively increases compared with affinities for κ light chains in other subgroups. Affinity for Vκ1 may relatively increase compared with affinity for κ light chains in one or more other subgroups. Examples of cases of improved binding selectivity for Vκ1 include cases in which affinity for Vκ1 increases and cases in which affinities for κ light chains in other subgroups decrease. Examples of κ light chains in subgroups other than subgroup 1 include Vκ3 and Vκ4. "Vκ3" refers to κ light chain subgroup 3; in other words, it refers to a light chain belonging to subgroup 3. "Vκ4" refers to κ light chain subgroup 4; in other words, it refers to a light chain belonging to subgroup 4. According to an embodiment of the present disclosure, the protein may be an immunoglobulin-binding protein with relatively increased affinity for Vκ1 compared with affinity for Vκ3 and/or Vκ4. According to an embodiment of the present disclosure, the protein may specifically be, for example, an immunoglobulin-binding protein that exhibits improved binding selectivity for Vκ1 as a result of reduced affinity for Vκ3 and/or Vκ4.

As a result of the improved binding selectivity for Vκ1, binding selectivity for immunoglobulins including Vκ1 may be improved. "Improved binding selectivity for immunoglobulins including Vκ1" may mean that affinity for immunoglobulins including Vκ1 relatively increases compared with affinities for immunoglobulins with lower percentages of Vκ1. For example, as a result of the improved binding selectivity for Vκ1, affinity for immunoglobulins including two Vκ1 chains may relatively increase compared with affinities for immunoglobulins including one Vκ1 chain and immunoglobulins including no Vκ1 chain. For example, as a result of the improved binding selectivity for Vκ1, affinity for immunoglobulins including one Vκ1 chain may relatively increase compared with affinity for immunoglobulins including no Vκ1 chain.

The amino acid substitution at a particular position is specifically at least one or more amino acid substitutions selected from Y42H (this notation denotes that the amino acid residue corresponding to tyrosine at position 42 of SEQ ID NO: 1 has been substituted with histidine; hereinafter, other amino acid substitutions are also to be interpreted in the same manner), K22E, K38D, K38P, Y42F, Y42W and N44P.

In other words, the amino acid substitution at a particular position is one or more amino acid substitutions selected from at least:
(1) Y42H;
(2) K22E;
(3) K38D or K38P;
(4) Y42F or Y42W; and
(5) N44P.
According to an embodiment of the present disclosure, therefore, the protein may have one or more amino acid substitutions selected from (1) to (5) above. According to an embodiment of the present disclosure, the protein may have, for example, one, two, three or four amino acid substitutions selected from the amino acid substitutions of (1) to (5) above. Of the amino acid substitutions of (1) to (5) above, however, amino acid substitutions at the same position are not selected simultaneously. For example, the amino acid substitution of (1) and the amino acid substitution of (4) are not selected simultaneously.

Of the amino acid substitutions of (1) to (5) above, the amino acid substitution of (1) above (Y42H), in particular, is an amino acid substitution with especially improved binding selectivity for immunoglobulins including a light chain belonging to subgroup 1 (Vκ1). An immunoglobulin-binding protein having at least the amino acid substitution of (1) above (Y42H), therefore, is a preferred example of a protein according to the present disclosure. That is, according to an embodiment of the present disclosure, the protein may have, for example, at least the amino acid substitution of (1) above (Y42H). The protein according to an embodiment of the present disclosure may specifically have, for example, the amino acid substitution of (1) above (Y42H) alone from among the amino acid substitutions of (1) to (5) above or may have a combination of the amino acid substitution of (1) above (Y42H) and one or more amino acid substitutions selected from the amino acid substitutions of (2), (3) and (5) (e.g., any of one, two or three).

In an embodiment, the amino acid substitution at a particular position may include one or more amino acid substitutions selected from (1) to (5) above.

According to an embodiment of the present disclosure, when the protein has two or more amino acid substitutions, the combination of these amino acid substitutions is not particularly restricted.

A form of a combination of amino acid substitutions selected from the amino acid substitutions of (1) to (5) above is the amino acid substitutions Y42H and K38D.

Another form of a combination of amino acid substitutions is a combination of one or more amino acid substitutions selected from at least (1) to (5) above and substitution(s) of one or more lysine (K) residues selected from at least (I) to (VII) below with a basic amino acid other than lysine (arginine (R) or histidine (H)) or an amino acid having a hydroxy group (serine (S), threonine (T) or tyrosine (Y)).
(I) The lysine residue corresponding to position 7 of SEQ ID NO: 1
(II) The lysine residue corresponding to position 13 of SEQ ID NO: 1
(III) The lysine residue corresponding to position 22 of SEQ ID NO: 1
(IV) The lysine residue corresponding to position 29 of SEQ ID NO: 1
(V) The lysine residue corresponding to position 38 of SEQ ID NO: 1
(VI) The lysine residue corresponding to position 48 of SEQ ID NO: 1
(VII) The lysine residue corresponding to position 67 of SEQ ID NO: 1

According to an embodiment of the present disclosure, the protein may have one, two, three, four, five, six or seven substitutions of these lysine residues.

However,
when the amino acid substitution of (III) above is selected, (2) the amino acid substitution K22E is replaced by the lysine residue substitution of (III) above, and
when the amino acid substitution of (V) above is selected, (3) the amino acid substitution K38E or K38P is replaced by the lysine residue substitution of (V) above.

Alternatively, of the amino acid substitutions of (I) to (VII) above, amino acid substitution(s) at the same position(s) as the one or more amino acid substitutions selected from (1) to (5) above may be excluded from selection. That is, for example, when at least (2) the amino acid substitution K22E is selected from the amino acid substitutions of (1) to (5) above, the amino acid substitution of (III) above may be excluded from selection.

The combination of amino acid substitutions, furthermore, is selected such that the one or more amino acids selected from at least the amino acid substitutions of (1) to (5) above are retained. That is, for example, when (2) the amino acid substitution K22E is selected alone from the amino acid substitutions of (1) to (5) above, the amino acid substitution of (III) above cannot be selected.

It should be noted that any lysine residue other than the lysine residue(s) substituted with a basic amino acid or an amino acid having a hydroxy group (i.e., of the lysine residues of (I) to (VII) above, any residue not selected) may be left unsubstituted (a lysine residue) or may be substituted with an amino acid that is not a basic amino acid or an amino acid having a hydroxy group. Examples of the latter include substitution of the lysine residue with glutamine or isoleucine or the above-described amino acid substitutions of (2) K22E and (3) K38D or K38P.

It should be noted that according to an embodiment of the present disclosure, the protein may further have at least one other amino acid substitution. An example of a form of such additional amino acid substitutions is the amino acid substitutions of <1> to <53> below. The amino acid substitutions of <1> to <53> below are amino acid substitutions that improve the alkaline stability of the immunoglobulin-binding protein. According to an embodiment of the present disclosure, the protein may have, for example, one, two, three, four, five, six, seven, eight or nine of the amino acid substitutions of <1> to <53> below or may have ten or more, as long as it retains immunoglobulin-binding activity. Of the amino acid substitutions of <1> to <53> below, however, amino acid substitutions at the same position are not selected simultaneously. For example, the amino acid substitution of <1> and the amino acid substitution of <18> are not selected simultaneously.
<1> N50Y
<2> E1G or E1V
<3> P3S
<4> E4K or E4G
<5> E5V
<6> K7A
<7> E8G
<8> E9V
<9> I17F
<10> G21R
<11> E27G or E27R
<12> K29P
<13> T31L
<14> T36A
<15> A41T
<16> N44S or N44G
<17> E49T or E49I
<18> Any of N50S, N50K or N50D
<19> G51V
<20> Any of E52V, E52G or E52D
<21> Y53F
<22> T54I or T54M
<23> Any of N62H, N62R, N62L, N62M or N62W
<24> N65Y
<25> A69T
<26> T2S or T2P
<27> P3R
<28> E5K or E5R
<29> Any of E27V, E27K or E27I
<30> F32L
<31> K38A
<32> N44I
<33> A47T or A47R
<34> E52N
<35> T2A
<36> E5D
<37> P6L or P6T
<38> K7P
<39> V14A
<40> I23R or I23V
<41> K29F
<42> T31M
<43> Any of E33D, E33G or E33V
<44> A35T
<45> T36S
<46> A37T
<47> A41I or A41Y
<48> N44H or N44R
<49> E52L or E52Y
<50> G60R
<51> I64L
<52> I66V
<53> A69L or A69V

According to an embodiment of the present disclosure, the protein may have one or more amino acid substitutions selected from (6) to (11) below in particular. According to an embodiment of the present disclosure, the protein may more particularly have the amino acid substitution of (6) below and one or more amino acid substitutions selected from (7) to (11) below.
(6) N50Y (this is the amino acid substitution of <1> above)
(7) E4G (this is one of the options for the amino acid substitution of <4>)
(8) K7A (this is the amino acid substitution of <6> above)
(9) E52L (this is one of the options for the amino acid substitution of <49>)
(10) Y53F (this is the amino acid substitution of <21> above)
(11) A69V (this is one of the options for the amino acid substitution of <53>)

An example of another embodiment of additional amino acid substitutions, furthermore, is the amino acid substitutions of <54> to <63> below. Immunoglobulin-binding proteins having the amino acid substitutions of <54> to <63> below allow for the elution of immunoglobulins (antibodies) bound thereto under milder pH conditions (i.e., conditions closer to neutral). According to an embodiment of the present disclosure, the protein may have, for example, one, two, three, four, five, six, seven, eight or nine of the amino acid substitutions of <54> to <64> below or may have all ten, as long as it retains immunoglobulin-binding activity.
<54> K7Q
<55> K13V
<56> K29V or K29I
<57> P6A
<58> E8R
<59> N15V
<60> I23F or I23L
<61> Any of E34D, E34F, E34L or E34V
<62> K38L
<63> N50M
<64> Y53S

According to an embodiment of the present disclosure, the protein may have the amino acid substitution of (12) below in particular.
(12) K29I (this is one of the options for the amino acid substitution of <56>)

However,
when the amino acid substitution of <31> or <62> above is selected, (3) the amino acid substitution K38E or K38P is replaced by the amino acid substitution of <31> or <62> above, and
when the amino acid substitution of any of <16>, <32> or <48> above is selected, (5) the amino acid substitution N44P is replaced by the amino acid substitution of any of <16>, <32> or <48> above.

Alternatively, of the amino acid substitutions of <1> to <64> above, amino acid substitution(s) at the same position(s) as the one or more amino acid substitutions selected from (1) to (5) above may be excluded from selection. That is, for example, when at least (3) the amino acid substitution K38E or K38P is selected from the amino acid substitutions of (1) to (5) above, the amino acid substitution of <31> or <62> above may be excluded from selection.

The combination of amino acid substitutions, furthermore, is selected such that the one or more amino acids selected from at least the amino acid substitutions of (1) to (5) above are retained. That is, for example, when (3) the amino acid substitution K38E or K38P is selected alone from the amino acid substitutions of (1) to (5) above, the amino acid substitution of <31> or <62> above cannot be selected.

Moreover, an example of yet another form of additional amino acid substitutions is the amino acid substitutions of <a> to <g> below. According to an embodiment of the present disclosure, the protein may have, for example, one, two or three of the amino acid substitutions of <a> to <d> below, as long as it retains immunoglobulin-binding activity. Of the amino acid substitutions of <a> to <g> below, however, amino acid substitutions at the same position are not selected simultaneously. For example, the amino acid substitution of <a> and the amino acid substitution of <c> are not selected simultaneously.
<a> E49D
<b> P6S
<c> E49V
<d> N62Y

Examples of other forms of combinations of amino acid substitutions include:
the amino acid substitutions K7A, K13R, K22R, K29I, K38E, Y42H, K48R and K67R;
the amino acid substitutions K7A, K13R, K22E, K29I, K38E, K48R and K67R;
the amino acid substitutions K7A, K13R, K22R, K29I, K38P, K48R and K67R;
the amino acid substitutions K7A, K13R, K22R, K29I, K38D, K48R and K67R;
the amino acid substitutions K7A, K13R, K22R, K29I, K38E, Y42F, K48R and K67R;
the amino acid substitutions K7A, K13R, K22R, K29I, K38E, Y42W, K48R and K67R;
the amino acid substitutions K7A, K13R, K22R, K29I, K38E, N44P, K48R and K67R; and
the amino acid substitutions K7A, K13R, K22R, K29I, K38D, Y42H, K48R and K67R.

Examples of other forms of combinations of amino acid substitutions include:
the amino acid substitutions E4G, P6S, K7A, K13R, K22R, K29I, K38E, Y42H, K48R, E49D, N50Y, G52L, Y53F, N62Y, K67R and A69V;
the amino acid substitutions E4G, P6S, K7A, K13R, K22E, K29I, K38E, K48R, E49D, N50Y, G52L, Y53F, N62Y, K67R and A69V;
the amino acid substitutions E4G, P6S, K7A, K13R, K22R, K29I, K38P, K48R, E49D, N50Y, G52L, Y53F, N62Y, K67R and A69V;
the amino acid substitutions E4G, P6S, K7A, K13R, K22R, K29I, K38D, K48R, E49D, N50Y, G52L, Y53F, N62Y, K67R and A69V;
the amino acid substitutions E4G, P6S, K7A, K13R, K22R, K29I, K38E, Y42W, K48R, E49D, N50Y, G52L, Y53F, N62Y, K67R and A69V;
the amino acid substitutions E4G, P6S, K7A, K13R, K22R, K29I, K38E, Y42F, K48R, E49D, N50Y, G52L, Y53F, N62Y, K67R and A69V;
the amino acid substitutions E4G, P6S, K7A, K13R, K22R, K29I, K38E, N44P, K48R, E49D, N50Y, G52L, Y53F, N62Y, K67R and A69V; and
the amino acid substitutions E4G, P6S, K7A, K13R, K22R, K29I, K38D, Y42H, K48R, E49D, N50Y, G52L, Y53F, N62Y, K67R and A69V.

According to an embodiment of the present disclosure further having one or more of the additional amino acid substitutions specified above (Namely, the amino acid substitutions of <1> to <64> above and <a> to <d> above. Hereinafter, "additional amino acid substitutions" is to be interpreted in the same manner), the protein may be one of the proteins specified below. These proteins are preferred in that they exhibit improved binding selectivity for antibodies having a particular light chain (e.g., Vκ1), and that their alkaline stability can also be improved:
An immunoglobulin-binding protein including the amino acid sequence set forth in SEQ ID NO: 1, provided that in the amino acid sequence, the protein has the amino acid substitutions E4G, P6S, K7A, K13R, K22R, K29I, K38E, Y42H, K48R, E49D, N50Y, G52L, Y53F, N62Y, K67R and A69V (an immunoglobulin-binding protein including the amino acid sequence set forth in SEQ ID NO: 100);
an immunoglobulin-binding protein including the amino acid sequence set forth in SEQ ID NO: 1, provided that in the amino acid sequence, the protein has the amino acid substitutions E4G, P6S, K7A, K13R, K22E, K29I, K38E, K48R, E49D, N50Y, G52L, Y53F, N62Y, K67R and A69V (an immunoglobulin-binding protein including the amino acid sequence set forth in SEQ ID NO: 95);
an immunoglobulin-binding protein including the amino acid sequence set forth in SEQ ID NO: 1, provided that in the amino acid sequence, the protein has the amino acid substitutions E4G, P6S, K7A, K13R, K22R, K29I, K38P, K48R, E49D, N50Y, G52L, Y53F, N62Y, K67R and A69V (an immunoglobulin-binding protein including the amino acid sequence set forth in SEQ ID NO: 96);
an immunoglobulin-binding protein including the amino acid sequence set forth in SEQ ID NO: 1, provided that in the amino acid sequence, the protein has the amino acid substitutions E4G, P6S, K7A, K13R, K22R, K29I, K38D, K48R, E49D, N50Y, G52L, Y53F, N62Y, K67R and A69V (an immunoglobulin-binding protein including the amino acid sequence set forth in SEQ ID NO: 97);
an immunoglobulin-binding protein including the amino acid sequence set forth in SEQ ID NO: 1, provided that in the amino acid sequence, the protein has the amino acid substitutions E4G, P6S, K7A, K13R, K22R, K29I, K38E, Y42W, K48R, E49D, N50Y, G52L, Y53F, N62Y, K67R and A69V (an immunoglobulin-binding protein including the amino acid sequence set forth in SEQ ID NO: 98);
an immunoglobulin-binding protein including the amino acid sequence set forth in SEQ ID NO: 1, provided that in the amino acid sequence, the protein has the amino acid substitutions E4G, P6S, K7A, K13R, K22R, K29I, K38E, Y42F, K48R, E49D, N50Y, G52L, Y53F, N62Y, K67R and A69V (an immunoglobulin-binding protein including the amino acid sequence set forth in SEQ ID NO: 99);
an immunoglobulin-binding protein including the amino acid sequence set forth in SEQ ID NO: 1, provided that in the amino acid sequence, the protein has the amino acid substitutions E4G, P6S, K7A, K13R, K22R, K29I, K38E, N44P, K48R, E49D, N50Y, G52L, Y53F, N62Y, K67R and A69V (an immunoglobulin-binding protein including the amino acid sequence set forth in SEQ ID NO: 101); and
an immunoglobulin-binding protein including the amino acid sequence set forth in SEQ ID NO: 1, provided that in the amino acid sequence, the protein has the amino acid substitutions E4G, P6S, K7A, K13R, K22R, K29I, K38D, Y42H, K48R, E49D, N50Y, G52L, Y53F, N62Y, K67R and A69V (an immunoglobulin-binding protein including the amino acid sequence set forth in SEQ ID NO: 102).

Herein, an amino acid sequence set forth in SEQ ID NO: 1 having one or more of the amino acid substitutions listed way of example above (i.e., the amino acid substitutions at particular positions and the additional amino acid substitutions specified above, which are optional) is also referred to as "SEQ ID NO: 1-derived substituted amino acid sequence." A SEQ ID NO: 1-derived substituted amino acid sequence is, in other words, an amino acid sequence set forth in SEQ ID NO: 1 in which one or more of the amino acid substitutions listed way of example above (i.e., the amino acid substitutions at particular positions and the additional amino acid substitutions specified above, which are optional) have occurred. In other words, furthermore, a SEQ ID NO: 1-derived substituted amino acid sequence is an amino acid sequence that is identical to the amino acid sequence set forth in SEQ ID NO: 1 except that it has one or more of the amino acid substitutions listed way of example above (i.e., the amino acid substitutions at particular positions and the additional amino acid substitutions specified above, which are optional). A SEQ ID NO: 1-derived substituted amino acid sequence is an example of a modified amino acid sequence as defined herein.

Variant sequences of modified amino acid sequences described by way of example above (e.g., SEQ ID NO: 1-derived substituted amino acid sequences) are also examples of modified amino acid sequences as defined herein. That is, according to an embodiment of the present disclosure, a protein including a variant sequence of a modified amino acid sequence as described by way of example above (e.g., a SEQ ID NO: 1-derived substituted amino acid sequence) and having immunoglobulin-binding activity is also an example of a protein. In the following, a case of a variant sequence of a SEQ ID NO: 1-derived substituted amino acid sequence will be described by way of example. This description, however, is also applicable to a variant sequence of any modified amino acid sequence.

The variant sequence of a SEQ ID NO: 1-derived substituted amino acid sequence is set such that the amino acid substitution at a particular position is retained (i.e., according to an embodiment of the present disclosure, such that the protein has the amino acid substitution at a particular position). The variant sequence of a SEQ ID NO: 1-derived substituted amino acid sequence, furthermore, may be set such that the additional amino acid substitution(s) described above (i.e., according to an embodiment of the present disclosure, such that the protein has the additional amino acid substitution(s) described above). Moreover, the variant sequence of a SEQ ID NO: 1-derived substituted amino acid sequence may additionally have, for example, one or more amino acid substitutions that are selected from the amino acid substitutions listed by way of example above (i.e., the amino acid substitutions at particular positions and the additional amino acid substitutions specified above, which are optional) and that the SEQ ID NO: 1-derived substituted amino acid sequence does not have. For example, when the SEQ ID NO: 1-derived substituted amino acid sequence does not have an additional amino acid substitution as specified above, the variant sequence of the SEQ ID NO: 1-derived substituted amino acid sequence may have the additional amino acid substitution.

An example of a variant sequence of a SEQ ID NO: 1-derived substituted amino acid sequence is a SEQ ID NO: 1-derived substituted amino acid sequence including one or several substitutions, deletions, insertions and/or additions of amino acid residues at one or several positions. That is, according to an embodiment of the present disclosure, the protein may further include one or several substitutions, deletions, insertions and/or additions of amino acid residues at one or several positions within the amino acid sequence set forth in SEQ ID NO: 1 in addition to one or more of the amino acid substitutions listed way of example above (i.e., the amino acid substitutions at particular positions and the additional amino acid substitutions specified above, which are optional), as long as it retains immunoglobulin-binding activity. That is, according to an embodiment of the present disclosure, a protein that includes the amino acid sequence set forth in SEQ ID NO: 1, provided that in the amino acid sequence, the protein has one or more of the amino acid substitutions listed way of example above (i.e., the amino acid substitutions at particular positions and the additional amino acid substitutions specified above, which are optional) and also includes one or several substitutions, deletions, insertions and/or additions of amino acid residues at one or several positions, while having immunoglobulin-binding activity is also an example of a protein. In other words, according to an embodiment of the present disclosure, the protein may be, for example, a protein including an amino acid sequence that is identical to SEQ ID NO: 1 except that it has one or more of the amino acid substitutions listed way of example above (i.e., the amino acid substitutions at particular positions and the additional amino acid substitutions specified above, which are optional) and also includes one or several substitutions, deletions, insertions and/or additions of amino acid residues at one or several positions, while having immunoglobulin-binding activity. It should be noted that the substitutions, deletions, insertions and/or additions of amino acid residues are selected such that the amino acid substitution at a particular position is retained. That is, the substitutions, deletions, insertions and/or additions of amino acid residues may occur at, for example, positions other than the particular position(s). The substitutions, deletions, insertions and/or additions of amino acid residues, furthermore, may be selected such that the additional amino acid substitution(s) is retained. That is, the substitutions, deletions, insertions and/or additions of amino acid residues may occur at, for example, positions other than the position(s) of the additional amino acid substitution(s). Moreover, the substitutions, deletions, insertions and/or additions of amino acid residues may include, for example, one or more amino acid substitutions that are selected from the amino acid substitutions listed by way of example above and that the SEQ ID NO: 1-derived substituted amino acid sequence does not have. The "one or several" refers specifically to, for example, any of 1 or more and 20 or fewer, 1 or more and 10 or fewer, 1 or more and 9 or fewer, 1 or more and 8 or fewer, 1 or more and 7 or fewer, 1 or more and 6 or fewer, 1 or more and 5 or fewer, 1 or more and 4 or fewer, 1 or more and 3 or fewer, 1 or more and 2 or fewer or 1, although depending partly on the positions of the amino acid residues within the three-dimensional structure of the protein and the types of the amino acid residues. An example of a substitution of an amino acid residue is a conservative substitution, in which substitution occurs between amino acids having similar physical properties and/or chemical properties. It is known to those skilled in the art that in the case of a conservative substitution, in general, the function of the protein is maintained between the protein in which the substitution has occurred and the protein in which the substitution has not occurred. An example of a conservative substitution is substitution between glycine and alanine, between serine and threonine or between glutamic acid and aspartic acid (Protein Structure and Function, Medical Sciences International, Ltd., 9, 2005). In addition, the substitutions, deletions, insertions and/or additions of amino acid residues also include, for example, those resulting from naturally occurring mutations (mutants or variants), such as those based on individual variations among or differences in species between the microorganisms from which the protein or the gene encoding it is derived.

Examples of variant sequences of SEQ ID NO: 1-derived substituted amino acid sequences, furthermore, also include an amino acid sequence having a high homology to a SEQ ID NO: 1-derived substituted amino acid sequence. That is, according to an embodiment of the present disclosure, a protein including an amino acid sequence having a high homology to an amino acid sequence set forth in SEQ ID NO: 1 having one or more of the amino acid substitutions listed by way of example above (i.e., the amino acid substitutions at particular positions and the additional amino acid substitution specified above, which are optional) while having immunoglobulin-binding activity is also an example of a protein. It should be noted that the changes in the amino acid sequence within such a range of homology are selected such that the amino acid substitution at a particular position is retained. That is, the changes in the amino acid sequence within such a range of homology may occur at, for example, positions other than the particular position(s). The changes in the amino acid sequence within such a range of homology, furthermore, may be selected such that the additional amino acid substitution(s) is retained. That is, the changes in the amino acid sequence within such a range of homology may occur at, for example, positions other than the position(s) of the additional amino acid substitution(s). Moreover, the changes in the amino acid sequence within such a range of homology may include, for example, one or more amino acid substitutions that are selected from the amino acid substitutions listed by way of example above and that the SEQ ID NO: 1-derived substituted amino acid sequence does not have. The "homology" may refer to similarity or identify, and may refer to identity in particular. "Homology to an amino acid sequence" refers to homology to the entire amino acid sequence. The "high homology" may refer to a homology of 70% or more, 80% or more, 90% or more or 95% or more. "Identity" between amino acid sequences refers to the percentage of amino acid residues that are of the same kind in both amino acid sequences (Experimental Medicine, February 2013, Vol. 31, No. 3, Yodosha Co., Ltd.). "Similarity" between amino acid sequences refers to the sum of the percentage of amino acid residues that are of the same kind in both amino acid sequences and the percentage of amino acid residues with side chains of similar character in both amino acid sequences (Experimental Medicine, February 2013, Vol. 31, No. 3, Yodosha Co., Ltd.). The amino acid residues with side chains of similar character are as described above. Homology between amino acid sequences can be determined using an alignment program, such as BLAST (Basic Local Alignment Search Tool) or FASTA.

Moreover, examples of modified amino acid sequences also include a variant sequence as described by way of example above in which the amino acid sequence also has one or more amino acid substitutions selected from the amino acid substitutions listed by way of example above (i.e., the amino acid substitutions at particular positions and the additional amino acid substitutions specified above, which are optional). For example, a modified amino acid sequence may be a variant sequence having at least the amino acid substitution at a particular position in which the amino acid sequence also has additional amino acid substitutions.

Examples of unmodified amino acid sequences, furthermore, also include a variant sequence of an unmodified amino acid sequence as described by way of example above (e.g., the amino acid sequence set forth in SEQ ID NO: 1). That is, examples of modified amino acid sequences also include a variant sequence of an unmodified amino acid sequence as described by way of example above (e.g., the amino acid sequence set forth in SEQ ID NO: 1) in which the amino acid sequence has one or more of the amino acid substitutions listed by way of example above (i.e., the amino acid substitutions at particular positions and the additional amino acid substitutions specified above, which are optional). That is, according to an embodiment of the present disclosure, a protein including a variant sequence of an unmodified amino acid sequence as described by way of example above (e.g., the amino acid sequence set forth in SEQ ID NO: 1), provided that in the variant sequence, the protein has one or more of the amino acid substitutions listed by way of example (i.e., the amino acid substitutions at particular positions and the additional amino acid substitutions specified above, which are optional), while having immunoglobulin-binding activity is also an example of a protein. In other words, according to an embodiment of the present disclosure, the protein may be a protein including, for example, an amino acid sequence that is identical to a variant sequence of an unmodified amino acid sequence as described by way of example above (e.g., the amino acid sequence set forth in SEQ ID NO: 1) except that it has one or more of the amino acid substitutions listed by way of example above. It should be noted that the variant sequence of an unmodified amino acid sequence as described by way of example above (e.g., the amino acid sequence set forth in SEQ ID NO: 1) may be actually present in a bacterium of the genus Finegoldia or may not. That is, "amino acid sequence of an immunoglobulin-binding domain of Protein L derived from a bacterium of the genus Finegoldia (amino acid sequence of an immunoglobulin-binding domain of FpL)" is not limited to amino acid sequences of immunoglobulin-binding domains of Protein L that are actually present in a bacterium of the genus Finegoldia; variant sequences of the amino acid sequences that are not actually present in a bacterium of the genus Finegoldia (i.e., hypothetical amino acid sequences) are also included. In the following, a case of a variant sequence of the amino acid sequence set forth in SEQ ID NO: 1 will be described by way of example. This description, however, is also applicable to a variant sequence of any unmodified amino acid sequence.

An example of a variant sequence of the amino acid sequence set forth in SEQ ID NO: 1 is an amino acid sequence set forth in SEQ ID NO: 1 in which the amino acid sequence includes one or several substitutions, deletions, insertions and/or additions of amino acid residues at one or several positions. That is, according to an embodiment of the present disclosure, a protein including the amino acid sequence set forth in SEQ ID NO: 1, provided that in the amino acid sequence, the protein includes one or several substitutions, deletions, insertions and/or additions of amino acid residues at one or several positions and also includes one or more of the amino acid substitutions listed by way of example above (i.e., the amino acid substitutions at particular positions and the additional amino acid substitutions specified above, which are optional), while having immunoglobulin-binding activity is also an example of a protein. The substitutions, deletions, insertions and/or additions of amino acid residues may occur at, for example, positions other than the particular position(s). The substitutions, deletions, insertions and/or additions of amino acid residues, furthermore, may occur at positions other than the position(s) of the additional amino acid substitution(s), for example.

Examples of variant sequences of the amino acid sequence set forth in SEQ ID NO: 1 also include an amino acid sequence having a high homology to the amino acid sequence set forth in SEQ ID NO: 1. That is, according to an embodiment of the present disclosure, a protein including an amino acid sequence having a high homology to the amino acid sequence set forth in SEQ ID NO: 1, provided that in the amino acid sequence (the amino acid sequence having a high homology to the amino acid sequence set forth in SEQ ID NO: 1), the protein has one or more of the amino acid substitutions listed by way of example above (i.e., the amino acid substitutions at particular positions and the additional amino acid substitution specified above, which are optional) while having immunoglobulin-binding activity is also an example of a protein. The changes in the amino acid sequence within such a range of homology may occur at, for example, positions other than the particular position(s). The changes in the amino acid sequence within such a range of homology, furthermore, may occur at positions other than the position(s) of the additional amino acid substitution(s), for example.

In other respects, the description of variant sequences of SEQ ID NO: 1-derived substituted amino acid sequences is applicable to variant sequences of the amino acid sequence set forth in SEQ ID NO: 1.

As used herein, "amino acid at position X of the amino acid sequence set forth in SEQ ID NO: 1" refers to the amino acid present at the Xth position as numbered from the N-terminus of the amino acid sequence set forth in SEQ ID NO: 1. The "amino acid residue corresponding to the amino acid at position X in the amino acid sequence set forth in SEQ ID NO: 1" in a particular amino acid sequence refers to the amino acid residue in the particular amino acid sequence that is located at the same position as the amino acid at position X of the amino acid sequence presented in SEQ ID NO: 1 in alignment between the particular amino acid sequence and the amino acid sequence of SEQ ID NO: 1. For example, in the case of the amino acid substitution Y42H, the "amino acid residue corresponding to tyrosine at position 42 of SEQ ID NO: 1" in a particular amino acid sequence refers to the amino acid residue in the particular amino acid sequence that is located at the same position as tyrosine at position 42 of the amino acid sequence presented in SEQ ID NO: 1 in alignment between the particular amino acid sequence and the amino acid sequence of SEQ ID NO: 1. It should be noted that "amino acid residue corresponding to the amino acid at position X of the amino acid sequence set forth in SEQ ID NO: 1" in the amino acid sequence set forth in SEQ ID NO: 1 refers to the amino acid at position X of the amino acid sequence set forth in SEQ ID NO: 1 itself. That is, the positions of the amino acid substitutions listed by way of example above (i.e., the amino acid substitutions at particular positions and, optionally, the additional amino acid substitutions) do not necessarily indicate absolute positions in the protein according to an embodiment of the present disclosure, but indicate relative positions based on the amino acid sequence set forth in SEQ ID NO: 1. That is, when a protein according to an embodiment of the present disclosure includes an insertion, deletion or addition of an amino acid residue at a position closer to the N-terminus than the position of an amino acid substitution as described by way of example above, the absolute position of this amino acid substitution can change according to it. According to an embodiment of the present disclosure, the positions of the amino acid substitutions listed by way of example above in the protein can be identified through, for example, alignment between the amino acid sequence of the protein according to an embodiment of the present disclosure and the amino acid sequence set forth in SEQ ID NO: 1. The alignment can be conducted by utilizing, for example, an alignment program, such as BLAST or FASTA. The same applies to the positions of the amino acid substitutions listed by way of example above in any amino acid sequence, such as a variant sequence of the amino acid sequence set forth in SEQ ID NO: 1. The amino acid residue before substitution in the amino acid substitutions listed by way of example above (i.e., the amino acid substitutions at particular positions and the additional amino acid substitutions specified above, which are optional), furthermore, indicates the kind of amino acid residue before substitution in the amino acid sequence set forth in SEQ ID NO: 1. In an unmodified amino acid sequence other than the amino acid sequence set forth in SEQ ID NO: 1, it may be conserved or may not.

According to an embodiment of the present disclosure, the protein may include solely one modified amino acid sequence or may include multiple modified amino acid sequences. According to an embodiment of the present disclosure, the protein may include, for example, two or more, three or more, four or more or five or more modified amino acid sequences, may include ten or fewer, seven or fewer, five or fewer, four or fewer, three or fewer or two or fewer modified amino acid sequences or may include any number of modified amino acid sequences that is a combination thereof that is not self-contradictory. According to an embodiment of the present disclosure, when the protein includes multiple modified amino acid sequences, the amino acid sequences of these multiple modified amino acid sequences may be identical or may not. These multiple modified amino acid sequences may be in a form in which they are directly connected (directly linked) or may be in a form in which they are connected by an appropriate linker (e.g., an oligopeptide composed of 5 or more and 25 or fewer amino acid residues).

According to an embodiment of the present disclosure, the protein may consist of a modified amino acid sequence or may further include an additional amino acid sequence. That is, according to an embodiment of the present disclosure, the protein may further include, for example, an additional amino acid sequence at its N-terminal side or C-terminal side. In other words, according to an embodiment of the present disclosure, the protein may have, for example, an additional amino acid sequence attached to the N-terminus or C-terminus of the modified amino acid sequence. An example of an additional amino acid sequence is an oligopeptide. Examples of oligopeptides that can be used as additional amino acid sequences include oligopeptides other than linkers as mentioned above. The additional amino acid sequence is, according to an embodiment of the present disclosure, not particularly restricted, unless it impairs the immunoglobulin-binding activity or stability of the protein. For example, the type and length of the additional amino acid sequence are, according to an embodiment of the present disclosure, not particularly restricted, unless the affinity for immunoglobulins or stability of the protein is impaired.

According to an embodiment of the present disclosure, the protein may include, for example, part of an additional immunoglobulin-binding domain in addition to the selected immunoglobulin-binding domain. For example, according to an embodiment of the present disclosure, when the protein includes a modified amino acid sequence of the C3 domain of FpL, the protein according to an embodiment of the present disclosure may further include part of the regions of FpL located closer to the N-terminus than the C3 domain (the C1 domain and the C2 domain) or may further include part of the region of FpL located closer to the C-terminus than the C3 domain (the C4 domain).

According to an embodiment of the present disclosure, the protein may include, for example, an oligopeptide that is useful for the purpose of specifically detecting or separating a target substance, at its N-terminal side or C-terminal side. Examples of such oligopeptides include polyhistidine and polyarginine. According to an embodiment of the present disclosure, furthermore, the protein may include, for example, an oligopeptide that is, according to an embodiment of the present disclosure, useful when immobilizing the protein on a solid phase, such as a support for chromatography, at its N-terminal side or C-terminal side. Examples of such oligopeptides include oligopeptides containing lysine or cysteine.

According to an embodiment of the present disclosure, when the protein includes an additional amino acid sequence as described above, the protein according to an embodiment of the present disclosure may be, for example, manufactured in a form that already includes the additional amino acid sequence or may have a separately manufactured additional amino acid sequence as described above attached thereto. According to an embodiment of the present disclosure, when the protein includes an additional amino acid sequence as described above, the protein according to an embodiment of the present disclosure can typically be manufactured by expressing it from a polynucleotide encoding the full-length amino acid sequence of the protein according to an embodiment of the present disclosure including the additional amino acid sequence as described above. That is, for example, a polynucleotide encoding the additional amino acid sequence and a polynucleotide encoding the protein according to an embodiment of the present disclosure (e.g., a protein that does not include the additional amino acid sequence) may be connected such that the additional amino acid sequence is attached to the N-terminus or C-terminus of the protein according to an embodiment of the present disclosure, and the protein according to an embodiment of the present disclosure may be expressed. Moreover, for example, a chemically synthesized additional amino acid sequence may be chemically bonded to the N-terminus or C-terminus of the protein according to an embodiment of the present disclosure (e.g., a protein that does not include the additional amino acid sequence).

According to an embodiment of the present disclosure, the protein can be manufactured by, for example, expressing it from a polynucleotide encoding the protein according to an embodiment of the present disclosure. Herein, a polynucleotide encoding a protein according to an embodiment of the present disclosure is also referred to as "polynucleotide according to an embodiment of the present disclosure." Specifically, a polynucleotide according to an embodiment of the present disclosure may be a polynucleotide including a nucleotide sequence encoding a protein according to an embodiment of the present disclosure.

The polynucleotide according to an embodiment of the present disclosure can be obtained through, for example, chemical synthesis or DNA amplification, for example by PCR. The DNA amplification can be conducted using, for example, a polynucleotide including the nucleotide sequence to be amplified, such as a nucleotide sequence encoding the protein, as the template. Examples of polynucleotides that can be used as templates include the genomic DNA of an organism expressing the protein according to an embodiment of the present disclosure, a cDNA for the protein according to an embodiment of the present disclosure and a vector including the polynucleotide according to an embodiment of the present disclosure.

The nucleotide sequence of the polynucleotide according to an embodiment of the present disclosure can be designed through, for example, conversion from the amino acid sequence of the protein according to an embodiment of the present disclosure. Conversion from the amino acid sequence into the nucleotide sequence can be performed using the standard codon table. It is, however, preferred to perform the conversion considering the codon usage frequency in the host to be transformed with the polynucleotide according to an embodiment of the present disclosure. As an example, when the host is Escherichia coli, AGA/AGG/CGG/CGA for arginine (R), ATA for isoleucine (I), CTA for leucine (L), GGA for glycine (G) and CCC for proline (P) are used with low frequency (codons commonly referred to as rare codons); therefore, the conversion may be performed such that these codons are avoided. The analysis of the codon usage frequency is also possible through the utilization of a public database (e.g., the Codon Usage Database, available at the website of Kazusa DNA Research Institute).

The polynucleotide according to an embodiment of the present disclosure may be obtained at once as the full-length sequence of the polynucleotide or may be obtained by acquiring polynucleotides consisting of partial sequences of the polynucleotide and then connecting them. Descriptions of methods for obtaining a polynucleotide according to an embodiment of the present disclosure such as those given above are not limited to when its full-length sequence is obtained at once; they are also applicable when its partial sequences are obtained.

It should be noted that the polynucleotide according to an embodiment of the present disclosure may be designed by connecting it to a polynucleotide encoding the amino acid sequence of any polypeptide so that it encodes a fused amino acid sequence.

The protein according to an embodiment of the present disclosure can be manufactured by, for example, culturing a recombinant host including a polynucleotide according to an embodiment of the present disclosure (hereinafter also referred to simply as "recombinant host according to an embodiment of the present disclosure") to allow the protein to be expressed and then recovering the expressed protein. The recombinant host according to an embodiment of the present disclosure can be obtained by, for example, transforming a host using a polynucleotide according to an embodiment of the present disclosure. The host is not particularly restricted as long as it is capable of expressing the protein according to an embodiment of the present disclosure by being transformed with the polynucleotide according to an embodiment of the present disclosure. Examples of hosts include animal cells, insect cells and microorganisms. Of these, examples of animal cells include COS cells, CHO (Chinese Hamster Ovary) cells, Hela cells, NIH3T3 cells and HEK293 cells, examples of insect cells include Sf9 cells and BTI-TN-5B1-4 cells, and examples of microorganisms include yeasts and bacteria. Examples of yeasts, furthermore, include yeasts of the genus Saccharomyces, such as Saccharomyces cerevisiae, yeasts of the genus Pichia, such as Pichia Pastoris, and yeasts of the genus Schizosaccharomyces, such as Schizosaccharomyces pombe, and examples of bacteria include bacteria of the genus Escherichia, such as Escherichia coli. Examples of Escherichia coli strains include JM109 and BL21(DE3). It should be noted that using yeast or Escherichia coli as the host is preferred in terms of productivity, and using Escherichia coli as the host is more preferred.

In the recombinant host according to an embodiment of the present disclosure, the polynucleotide according to an embodiment of the present disclosure only needs to be retained in a manner that allows its expression. Specifically, the polynucleotide according to an embodiment of the present disclosure only needs to be retained such that it is expressed under regulation of a promoter that functions in the host. When the host is Escherichia coli, examples of promoters that function in the host include the trp promoter, the tac promoter, the trc promoter, the lac promoter, the T7 promoter, the recA promoter and the lpp promoter.

In the recombinant host according to an embodiment of the present disclosure, furthermore, the polynucleotide according to an embodiment of the present disclosure may be present on a vector that autonomously replicates outside the genomic DNA. That is, the host may be transformed with an expression vector including the polynucleotide according to an embodiment of the present disclosure (hereinafter also referred to simply as "expression vector according to an embodiment of the present disclosure") to produce the recombinant host according to an embodiment of the present disclosure. The expression vector according to an embodiment of the present disclosure is obtained by, for example, inserting the polynucleotide according to an embodiment of the present disclosure into an expression vector at an appropriate position. It should be noted that the expression vector is not particularly restricted as long as it can stably exist and replicate within the host to be transformed. When the host is Escherichia coli, examples of expression vectors include a pET plasmid vector, a pUC plasmid vector and a pTrc plasmid vector. In addition, the expression vector according to an embodiment of the present disclosure may include a selection marker, such as an antibiotic resistance gene. The appropriate position, furthermore, refers to a position at which the insertion does not disrupt regions responsible for the replicating function, selection marker or transferability of the expression vector. When the polynucleotide according to an embodiment of the present disclosure is inserted into the expression vector, it is preferred to insert the polynucleotide in a state in which it is linked to a functional polynucleotide, such as the promoter required for expression.

Moreover, in the recombinant host according to an embodiment of the present disclosure, the polynucleotide according to an embodiment of the present disclosure may be introduced into the genomic DNA. The introduction of the polynucleotide according to an embodiment of the present disclosure into the genomic DNA can be conducted by utilizing, for example, gene transfer by homologous recombination. Examples of methods for gene transfer by homologous recombination include methods in which linearchain DNA is used, such as Red-driven integration (Datsenko, K.A, and Wanner, B.L., Proc. Natl. Acad. Sci. USA., 2000; 97: 6640-6645), methods in which a vector including a temperature-sensitive origin of replication is used, methods in which a vector having no origin of replication that functions within the host and transduction, in which a phage is used.

The transformation of the host using the polynucleotide, such as that in an expression vector according to an embodiment of the present disclosure, can be conducted by, for example, methods commonly used by those skilled in the art. For example, when Escherichia coli is selected as the host, the transformation can be performed by methods such as the competent cell technique, the heat shock technique and electroporation. By performing screening for host cells containing the polynucleotide according to an embodiment of the present disclosure by an appropriate method after transformation, the recombinant host according to an embodiment of the present disclosure can be obtained.

It should be noted that information regarding gene engineering methods, such as expression vectors and promoters that can be utilized in various microorganisms, can be obtained through the use of known publications, such as "Fundamental Microbiology 8: Genetic Engineering, Kyoritsu Shuppan Co., Ltd. (1987)."

When the recombinant host according to an embodiment of the present disclosure contains an expression vector according to an embodiment of the present disclosure, the expression vector according to an embodiment of the present disclosure can be prepared from the recombinant host according to an embodiment of the present disclosure. For example, a culture obtained by culturing the recombinant host according to an embodiment of the present disclosure can be treated using alkali extraction or a commercially available extraction kit, such as a QIAprep Spin Miniprep kit (manufactured by QIAGEN N.V.), to prepare the expression vector according to an embodiment of the present disclosure.

By culturing the recombinant host according to an embodiment of the present disclosure, the protein according to an embodiment of the present disclosure can be expressed. By culturing the recombinant host to allow the protein to be expressed and recovering the expressed protein, furthermore, the protein can be manufactured. That is, the present description discloses a method for manufacturing a protein as described above, the method comprising a step of culturing a recombinant host as described above to allow the protein to be expressed and a step of recovering the expressed protein. The medium composition and the culture conditions can be set as appropriate according to conditions such as the type of the host and the characteristics of the protein. The medium composition and the culture conditions can be set to conditions under which, for example, the host can grow and is capable of expressing the protein. The medium can be, for example, a medium containing a carbon source, a nitrogen source, an inorganic salt and various other organic components and inorganic components as appropriate. Specifically, when the host is Escherichia coli, an example of a preferred medium is LB (Luria-Bertani) medium (1% (w/v) tryptone, 0.5% (w/v) yeast extract and 1% (w/v) NaCl) supplemented with necessary nutrition sources. Incidentally, it is preferred to add an antibiotic corresponding to an antibiotic resistance gene included in the expression vector according to an embodiment of the present disclosure to the medium before culturing, so that the recombinant host according to an embodiment of the present disclosure is selectively grown depending on whether or not the expression vector has been introduced. For example, when the expression vector includes the kanamycin resistance gene, kanamycin can be added to the medium. The same applies when the polynucleotide according to an embodiment of the present disclosure has been introduced into the genomic DNA. The medium, furthermore, may contain one or more reducing agents selected from the group consisting of glutathione, cysteine, cystatin, thioglycolate and dithiothreitol. Moreover, the medium may contain a reagent that promotes protein secretion from the transformant into the culture broth, such as glycine. For example, when the host is Escherichia coli, it is preferred to add 2% (w/v) or less glycine to the medium. The culturing temperature is, for example, generally 10°C or above and 40°C or below, preferably 20°C or above and 37°C or below, more preferably approximately 25°C, when the host is Escherichia coli. The pH of the medium is, for example, pH 6.8 or above and pH 7.4 or below, preferably approximately pH 7.0, when the host is Escherichia coli. When the protein is expressed under regulation of an inducible promoter, furthermore, it is preferred to apply induction such that the protein can be successfully expressed. The induction of expression can be performed using, for example, an inducer selected according to the type of the promoter. An example of an inducer is IPTG (Isopropyl-β-D-thiogalactopyranoside). When the host is Escherichia coli, the expression of the protein can be induced by, for example, adding an appropriate amount of IPTG when the turbidity (absorbance at 600 nm) of the culture broth reaches approximately 0.5 to 1.0 and continuing culturing. The concentration of IPTG added is, for example, 0.005 mM or more and 1.0 mM or less, preferably 0.01 mM or more and 0.5 mM or less. The induction of expression, such as induction with IPTG, can be performed under, for example, conditions well known in the technical field.

The protein according to an embodiment of the present disclosure can be separated from the culture and recovered by a method suitable for its form of expression. It should be noted that as used herein, "culture" refers to the entire culture broth obtained through culturing or a portion thereof. This portion is not particularly restricted as long as it is a portion containing the protein. Examples of the portion include cultured cells of the transformant according to an embodiment of the present disclosure and the medium after culturing (i.e., the culture supernatant). For example, when the protein accumulates in the culture supernatant, the cells can be separated through a centrifugation operation, and the protein can be recovered from the resulting culture supernatant. When the protein accumulates within the cells (including the periplasm), furthermore, the cells harvested through a centrifugation operation can then be disrupted by adding, for example, an enzymatic treatment agent or a surfactant, and the protein can be recovered from the disrupted cells. The recovery of the protein from the culture supernatant or disrupted cells as described above can be conducted by, for example, known methods used for separation and purification of proteins. Examples of recovery methods include ammonium sulfate fractionation, ion-exchange chromatography, hydrophobic interaction chromatography, affinity chromatography, gel filtration chromatography and isoelectric precipitation.

The protein according to an embodiment of the present disclosure can be used for, for example, separation or analysis of immunoglobulins (antibodies). When used for this purpose, the protein can be used in the form of, for example, an immunoglobulin adsorbent comprising an insoluble support and the protein immobilized on the insoluble support (hereinafter also referred to simply as "immunoglobulin adsorbent according to an embodiment of the present disclosure"). It should be noted that as used herein, "separation of immunoglobulins" is not limited to the separation of immunoglobulins from a solution in which impurities coexist; separation between immunoglobulins based on, for example, their structure, properties or activity is also included. The insoluble support is not particularly restricted as long as it is a support insoluble in aqueous solutions. Examples of insoluble supports include supports made from polysaccharides, such as agarose, alginates (alginic acid salts), carrageenan, chitin, cellulose, dextrin, dextran and starch, supports made from synthetic polymers, such as polyvinyl alcohol, polymethacrylates, poly(2-hydroxyethyl methacrylate) and polyurethane, and supports made from ceramics, such as silica. Of these, supports made from polysaccharides and supports made from synthetic polymers are particularly preferred as insoluble supports. Examples of such preferred supports include polymethacrylate gels into which hydroxy groups have been introduced, such as TOYOPEARL (manufactured by Tosoh Corporation), agarose gels, such as Sepharose (manufactured by Cytiva), and cellulose gels, such as Cellufine (manufactured by JNC Corporation). The shape of the insoluble support is not particularly restricted. The insoluble support may be in a shape that can be, for example, packed in a column. The insoluble support may be, for example, a granular material or a nongranular material. The insoluble support, furthermore, may be porous or nonporous, for example.

The immunoglobulins (antibodies) under separation or analysis according to an embodiment of the present disclosure are not particularly restricted as long as the protein according to an embodiment of the present disclosure has affinity for them. Specifically, the immunoglobulins may be ones having κ light chains. More specifically, the immunoglobulins may be ones having Vκ1. The immunoglobulins may have κ light chains in other subgroups in addition to Vκ1. Examples of κ light chains in subgroups other than Vκ1 include Vκ3 and Vκ4. The immunoglobulins may include other regions, such as an Fc region, in addition to the κ light chains or may not. The immunoglobulins may be ones lacking an Fc region, such as single-chain Fv (scFv), Fab, F(ab')₂, IgA or bispecific T cell-engaging (BiTE) antibodies. The immunoglobulins may be monoclonal antibodies or may be polyclonal antibodies. The immunoglobulins may be any of, for example, IgG, IgM, IgA, IgD or IgE. IgG may be, for example, any of IgG1, IgG2, IgG3 or IgG4. The source of the immunoglobulins is not particularly restricted. The immunoglobulins may be ones derived from a single organism species or may be ones derived from a combination of two or more species of organisms. Examples of immunoglobulins include chimeric antibodies, humanized antibodies, human antibodies and variants (such as amino acid substitution variants) thereof. Antibodies having an artificially modified structure, such as bispecific antibodies, fused antibodies composed of κ light chains and another protein and conjugates of κ light chains and a drug (ADC), are also examples of immunoglobulins. It should be noted that "immunoglobulins" also includes antibody drugs. A particularly notable example of immunoglobulins is bispecific antibodies. Examples of bispecific antibodies include trifunctional antibodies, F(ab')₂, single-chain Fv (scFv) and bispecific T cell-engaging (BiTE) antibodies. The bispecific antibodies may have Vκ1 and Vκ3 or may have Vκ1 and Vκ4. The bispecific antibodies may have Vκ1 and Vκ3 in particular. By utilizing the protein according to an embodiment of the present disclosure, immunoglobulins including Vκ1, for example, can be separated from immunoglobulins with higher or lower percentages of Vκ1. Specifically, by utilizing the protein, immunoglobulins including two Vκ1 chains, for example, can be separated from immunoglobulins including one Vκ1 chain and immunoglobulins including no Vκ1 chain. Specifically, by utilizing the protein, immunoglobulins including one Vκ1 chain (e.g., bispecific antibodies having Vκ1 and a κ light chain in another subgroup), for example, can be separated from immunoglobulins including two Vκ1 chains or immunoglobulins including no Vκ1 chain.

The protein according to an embodiment of the present disclosure may be immobilized onto the insoluble support through, for example, covalent bonding. As a specific example, by covalently bonding the protein and the insoluble support via active groups that the insoluble support has, the protein can be immobilized onto the insoluble support. That is, the insoluble support may have active groups, for example on its surface. Examples of such active groups include N-hydroxysuccinimide (NHS)-activated ester groups, epoxy groups, carboxy groups, maleimide groups, haloacetyl groups, tresyl groups, formyl groups and haloacetamides. The insoluble support having active groups may be, for example, a commercially available insoluble support having active groups used as is or may be an insoluble support into which active groups have been introduced. Examples of commercially available supports having active groups include TOYOPEARL AF-Epoxy-650M and TOYOPEARL AF-Tresyl-650M (both manufactured by Tosoh Corporation), HiTrap NHS-activated HP Columns, NHS-activated Sepharose 4 Fast Flow and Epoxyactivated Sepharose 6B (all manufactured by Cytiva) and SulfoLink Coupling Resin (manufactured by Thermo Fisher Scientific Inc.).

An example of a method for introducing active groups onto the surface of the support is the method of allowing one of compounds having two or more active sites to react with, for example, hydroxy groups, epoxy groups, carboxy groups or amino groups present on the surface of the support.

Examples of compounds that introduce epoxy groups to hydroxy groups or amino groups present on the surface of the support include epichlorohydrin, ethanediol diglycidyl ether, butanediol diglycidyl ether and hexanediol diglycidyl ether.

Examples of compounds that introduce carboxy groups to epoxy groups present on the surface of the support, furthermore, include 2-mercaptoacetic acid, 3-mercaptopropionic acid, 4-mercaptobutyric acid, 6-mercaptobutyric acid, glycine, 3-aminopropionic acid, 4-aminobutyric acid and 6-aminohexanoic acid.

Moreover, examples of compounds that introduce maleimide groups to hydroxy groups, epoxy groups, carboxy groups or amino groups present on the surface of the support include N-(ε-maleimidocaproic acid)hydrazide, N-(ε-maleimidopropionic acid)hydrazide, 4-(4-N-maleimidophenyl)acetic acid hydrazide, 2-aminomaleimide, 3-aminomaleimide, 4-aminomaleimide, 6-aminomaleimide, 1-(4-aminophenyl)maleimide, 1-(3-aminophenyl)maleimide, 4-(maleimido)phenyl isocyanate, 2-maleimidoacetic acid, 3-maleimidopropionic acid, 4-maleimidobutyric acid, 6-maleimidohexanoic acid, N-(α-maleimidoacetoxy)succinimide ester, (m-maleimidobenzoyl) N-hydroxysuccinimide ester, succinimidyl-4-(maleimidomethyl)cyclohexane-1-carbonyl-(6-aminohexanoic acid), succinimidyl-4-(maleimidomethyl)cyclohexane-1-carboxylic acid, (p-maleimidobenzoyl) N-hydroxysuccinimide ester and (m-maleimidobenzoyl) N-hydroxysuccinimide ester.

Examples of compounds that introduce haloacetyl groups to hydroxy groups or amino groups present on the surface of the support, furthermore, include chloroacetic acid, bromoacetic acid, iodoacetic acid, chloroacetyl chloride, bromoacetyl chloride, bromoacetyl bromide, chloroacetic anhydride, bromoacetic anhydride, iodoacetic anhydride, 2-(iodoacetamido)acetic acid-N-hydroxysuccinimide ester, 3-(bromoacetamido)propionic acid-N-hydroxysuccinimide ester and 4-(iodoacetyl)aminobenzoic acid-N-hydroxysuccinimide ester.

In addition, the method of allowing an ω-alkenylalkane glycidyl ether to react with hydroxy groups or amino groups present on the surface of the support and then halogenating the ω-alkenyl moiety with a halogenating agent to activate it is also an example of a method for introducing active groups onto the surface of the support. Examples of ω-alkenylalkane glycidyl ethers include allyl glycidyl ether, 3-butenyl glycidyl ether and 4-pentenyl glycidyl ether. Examples of halogenating agents include N-chlorosuccinimide, N-bromosuccinimide and N-iodosuccinimide.

The method of introducing the active groups to carboxy groups present on the surface of the support using a condensing agent and an additive, furthermore, is also an example of a method for introducing active groups onto the surface of the support. Examples of condensing agents include 1-ethyl-3-(3-dimethylaminopropyl)carbodiimide (EDC), dicyclohexylcarbodiamide and carbonyldiimidazole. Examples of additives include N-hydroxysuccinimide (NHS), 4-nitrophenol and 1-hydroxybenztriazole.

The immobilization of the protein according to an embodiment of the present disclosure onto the insoluble support can be conducted in, for example, a buffer. Examples of buffers include acetate buffer, phosphate buffer, MES (2-Morpholinoethanesulfonic acid) buffer, HEPES (4-(2-Hydroxyethyl)-1-piperazineethanesulfonic acid) buffer, Tris buffer and borate buffer. The reaction temperature during the immobilization can be set as appropriate, for example according to conditions such as the reactivity of the active groups and the stability of the protein according to an embodiment of the present disclosure. The reaction temperature during the immobilization may be, for example, 5°C or above and 50°C or below, preferably is 10°C or above and 35°C or below.

The immunoglobulin adsorbent according to an embodiment of the present disclosure can be used for, for example, separation of immunoglobulins (antibodies) by configuring the adsorbent as a column packed with it. For example, by applying a solution containing immunoglobulins to a column packed with the immunoglobulin adsorbent to allow the immunoglobulins to be adsorbed onto the adsorbent and eluting the immunoglobulins adsorbed on the adsorbent, the immunoglobulins can be separated. That is, the present description discloses a method for separating an immunoglobulin contained in a solution, the method comprising a step of applying a solution containing the immunoglobulin to a column packed with the adsorbent described above to allow the immunoglobulin to be adsorbed onto the adsorbent and a step of eluting the immunoglobulin adsorbed on the adsorbent. The solution containing immunoglobulins can be applied to the column using, for example, liquid delivery means, such as a pump. It should be noted that the solution containing immunoglobulins may be subjected to prior solvent replacement using an appropriate buffer before application to the column. Before the application of the solution containing immunoglobulins to the column, furthermore, the column may be equilibrated using an appropriate buffer. Through the equilibration of the column, it is expected that the immunoglobulins can be, for example, separated with higher purity. Examples of buffers that can be used for solvent replacement or equilibration include phosphate buffer, acetate buffer and MES buffer. To such a buffer, 1 mM or more and 1000 mM or less of sodium chloride or another inorganic salt, for example, may be further added. The buffer used for solvent replacement and the buffer used for equilibration may be the same or may not be the same. When components other than immunoglobulins, such as impurities, remain in the column after the passage of the solution containing immunoglobulins through the column, furthermore, such components may be removed from the column before the elution of the immunoglobulins adsorbed on the immunoglobulin adsorbent. The components other than immunoglobulins can be removed from the column using, for example, an appropriate buffer. For the buffer used for the removal of components other than immunoglobulins, the descriptions of the buffers used for solvent replacement or equilibration are applicable. The immunoglobulins adsorbed on the immunoglobulin adsorbent can be eluted by, for example, weakening the interactions between the immunoglobulins and the ligand (the protein according to an embodiment of the present disclosure). Examples of means for weakening the interactions between the immunoglobulins and the ligand (the protein according to an embodiment of the present disclosure) include a change in pH, addition of a counter peptide, temperature increase and a change in salt concentration. A particularly notable example of means for weakening the interactions between the immunoglobulins and the ligand (the protein according to an embodiment of the present disclosure) is a change in pH. That is, a particularly notable example of the step of eluting the immunoglobulin adsorbed on the adsorbent is a step of eluting the immunoglobulin adsorbed on the adsorbent through a change in pH. An example of a change in pH is a decrease in pH. An example of a decrease in pH is a decrease in pH caused using a buffer. Specifically, the immunoglobulins adsorbed on the immunoglobulin adsorbent can be eluted using, for example, an appropriate eluent. An example of an eluent is a buffer that is more acidic than the buffers used for solvent replacement and equilibration. Examples of such acidic buffers include citrate buffer, glycine hydrochloride buffer and acetate buffer. That is, by utilizing the acidic buffer, the pH can be reduced. The pH of the eluent can be set within a range in which, for example, the functions of the immunoglobulins (e.g., affinity for antigens) are not impaired. The pH of the eluent may be, for example, 2 or higher, 2.1 or higher, 2.2 or higher, 2.3 or higher, 2.4 or higher, 2.5 or higher, 2.6 or higher, 2.7 or higher, 2.8 or higher, 2.9 or higher, 3 or higher, 3.1 or higher or 3.2 or higher, may be 4 or lower, 3.5 or lower, 3.2 or lower, 3.1 or lower, 3 or lower, 2.9 or lower, 2.8 or lower, 2.7 or lower, 2.6 or lower or 2.5 or lower or may be a combination thereof that is not self-contradictory. Specifically, the pH of the eluent may be 2 or higher and 4 or lower, 2.2 or higher and 4 or lower or 2.4 or higher and 4 or lower.

By separating immunoglobulins (antibodies) by the method described above, separated immunoglobulins are obtained. That is, the method for separating an immunoglobulin may be, in an aspect thereof, a method for manufacturing an immunoglobulin, and, specifically, may be a method for manufacturing a separated immunoglobulin. The immunoglobulins are obtained as, for example, an eluted fraction containing the immunoglobulins. That is, a fraction containing eluted immunoglobulins can be collected. The collection of the fraction can be performed by, for example, ordinary methods. Examples of methods for collecting the fraction include the method of replacing the collection vessel at regular time intervals or regular volume intervals, the method of changing the collection vessel according to the shape of the chromatogram of the eluate and the method of collecting fractions using, for example, an automatic fraction collector, such as an autosampler. The recovery of the immunoglobulins from the fraction containing the immunoglobulins, furthermore, is also possible. The recovery of the immunoglobulins from the fraction containing the immunoglobulins can be performed by, for example, known methods used for separation and purification of proteins.

### EXAMPLES

Some embodiments of the present disclosure will now be more specifically described using examples and a comparative example. The present disclosure, however, is not limited to these examples. It should be noted that the reference example is not illustrative of embodiments of the present disclosure.

### EXAMPLE 1 Preparation of an Expression Vector for the C3 Immunoglobulin-Binding Domain of Protein L

(1) A polynucleotide (SEQ ID NO: 2) encoding the amino acid sequence of the C3 immunoglobulin-binding domain (the amino acid residues at positions 394 to 463 of GenBank No. AAA67503 (SEQ ID NO: 106), hereinafter also denoted as "FpL_C3") of Protein L derived from a bacterium of the genus Finegoldia (Finegoldia magna) (hereinafter FpL), which consists of the amino acid sequence set forth in SEQ ID NO: 1, was synthesized. It should be noted that during the synthesis, a recognition sequence for the restriction enzyme NcoI (5'-CCATGG-3') was attached to the 5'-end, and a nucleotide sequence encoding six histidine residues (5'-CATCACCACCATCACCAC-3'; SEQ ID NO: 107), a stop codon and a recognition sequence for the restriction enzyme HindIII (5'-AAGCTT-3') were attached to the 3'-end.
(2) The polynucleotide obtained through the synthesis, which included SEQ ID NO: 2, was treated with the restriction enzymes NcoI and HindIII, and then the band having the size of the desired product was confirmed by agarose gel electrophoresis. After excision of the band of interest, the polynucleotide was purified using a QIAquick Gel Extraction kit (manufactured by QIAGEN N.V.).
(3) The polynucleotide obtained in (2) and a plasmid pET-26b predigested with the restriction enzymes NcoI and HindIII were ligated using a DNA Ligation Kit (manufactured by Takara Bio Inc.). The Escherichia coli strain BL21(DE3) was transformed using the ligation product, and the transformant was cultured on an LB (Luria-Bertani) plate medium containing 50 µg/mL kanamycin (37°C, 16 hours).
(4) The transformant (recombinant Escherichia coli) obtained in (3) was subjected to selection, and the selected cells were cultured in an LB medium containing 50 µg/mL kanamycin and then purified using a QIAprep Spin Miniprep kit (manufactured by QIAGEN N.V.). In this manner, a vector (designated as pET-FpL_C3) capable of expressing FpL_C3 was obtained.
(5) Of the expression vector pET-FpL_C3 obtained in (4), the polynucleotide encoding FpL_C3 and its flanking regions were subjected to a cycle sequencing reaction using a Big Dye Terminator Cycle Sequencing ready Reaction kit (manufactured by Thermo Fisher Scientific Inc.), which is based on the chain terminator method. Then the nucleotide sequence was analyzed using the ABI Prism 3700 DNA analyzer, a fully automatic DNA sequencer (manufactured by Thermo Fisher Scientific Inc.). It should be noted that during the analysis, oligonucleotides consisting of the sequence set forth in SEQ ID NO: 3 (5'-TAATACGACTCACTATAGGG-3') or SEQ ID NO: 4 (5'-TATGCTAGTTATTGCTCAG-3') were used as sequencing primers.

As a result of the sequence analysis, it was confirmed that a polynucleotide consisting of the sequence set forth in SEQ ID NO: 2 had been inserted into the expression vector pET-FpL_C3.

### EXAMPLE 2 Mutagenesis of FpL_C3 and Library Construction

### (Part 1)

Mutations were randomly introduced by error-prone PCR into the immunoglobulin-binding protein-encoding polynucleotide portion (SEQ ID NO: 2) of the expression vector pET-FpL_C3 prepared in Example 1.

(1) Using the pET-FpL_C3 prepared in Example 1 as the template DNA, error-prone PCR was performed. The error-prone PCR was performed by preparing a reaction solution having the composition presented in Table 1, then subjecting the reaction solution to heat treatment at 95°C for 2 minutes, carrying out 30 cycles of reaction, in which a first step at 95°C for 30 seconds, a second step at 50°C for 30 seconds and a third step at 72°C for 90 seconds constituted one cycle, and finally applying heat treatment at 72°C for 7 minutes. As a result of the error-prone PCR, mutations were successfully introduced into the polynucleotide (SEQ ID NO: 2) encoding FpL_C3. The average mutation introduction rate was 1.6%.

### [Table 1]

**Table 1**

| Composition | Volume |
|---|---|
| 10 ng/µL template DNA | 1 µL |
| 2.5 mM dNTP mixture | 8 µL |
| 10 µM PCR forward primer (SEQ ID NO: 3) | 4 µL |
| 10 µM PCR reverse primer (SEQ ID NO: 4) | 4 µL |
| 10 mM MnCl₂ | 2 µL |
| 10 mM MgCl₂ | 12 µL |
| Go Taq DNA polymerase (manufactured by Promega Corporation) | 1 µL |
| H₂O | Up to 100 µL |

(2) After purification, the resulting PCR product was digested with the restriction enzymes NcoI and HindIII. The digested product was ligated into the expression vector pET-26b predigested with the restriction enzymes NcoI and HindIII.

(3) After completion of the ligation reaction, the Escherichia coli strain BL21(DE3) was transformed using the reaction solution, and the transformants were cultured on an LB plate medium containing 50 µg/mL kanamycin (37°C, 16 hours). Then the colonies that formed on the plate were defined as a random mutant library.

### EXAMPLE 3 Screening of FpL_C3 Amino Acid Substitution Mutants

(1) The random mutant library (transformants) constructed in Example 2 or the transformant prepared in Example 1, expressing FpL_C3 (SEQ ID NO: 1), was inoculated into 250 µLof 2× YT liquid medium (1.6% (w/v) tryptone, 1% (w/v) yeast extract and 0.5% (w/v) sodium chloride) containing 50 µg/mL kanamycin and shake-cultured overnight at 37°C using a 96-deep-well plate.
(2) After culturing, 5 µL of the culture broth in (1) was transferred into 500 µL of 2× YT liquid medium containing 50 µg/mL kanamycin, 0.3% (w/v) glycine and 0.01 mM IPTG (Isopropyl-β-D-thiogalactopyranoside), and the cells were further shake-cultured overnight at 20°C using a 96-deep-well plate.
(3) After culturing, the culture supernatant was obtained through a centrifugation operation and mixed with an equal volume of 1.0 M NaOH, and the resulting mixture was subjected to alkali treatment at 38°C for 15 minutes.
(4) The antibody-binding activity of the immunoglobulin-binding protein after the alkali treatment was measured by ELISA as described below, and the remaining activity was calculated by dividing the antibody-binding activity of the immunoglobulin-binding protein after the alkali treatment in (3) by the antibody-binding activity of the immunoglobulin-binding protein without the alkali treatment in (3).
(4-1) A human antibody (gamma-globulin preparation, manufactured by The Chemo-Sero-Therapeutic Research Institute) prepared to 10 µg/mL using Tris-Buffered Saline (TBS) was dispensed into each well of a 96-well microplate and immobilized (4°C, 16 hours). After immobilization, blocking was performed using skim milk (manufactured by Becton, Dickinson and Company) prepared to 2% (w/v) using TBS.
(4-2) Each well of the 96-well microplate was washed with a washing buffer (0.05 M Tris buffer (pH 7.5) containing 0.15 M NaCl and 0.05% (w/v) Tween 20 (trade name), hereinafter also referred to as "washing buffer A"). Then a solution containing the immunoglobulin-binding protein under evaluation for antibody-binding activity was added, and the immunoglobulin-binding protein and the immobilized gamma-globulin were allowed to react (30°C, 1 hour).
(4-3) After completion of the reaction, the wells were washed with washing buffer A, and 100 µL/well of Anti-6-His Antibody (manufactured by BETHYL LABORATORIES, Inc.), diluted to 100 ng/mL, was added and allowed to react (30°C, 1 hour).
(4-4) After completion of the reaction, the wells were washed with washing buffer A, and 50 µL/well of TMB Peroxidase Substrate (manufactured by KPL, Inc.) was added. Then the reaction was terminated by adding 50 µL/well of 1 M phosphoric acid, and the absorbance at 450 nm was measured using a microplate reader (manufactured by Tecan Trading AG) .
(5) Approximately 1600 strains of transformants were evaluated, and transformants expressing immunoglobulin-binding proteins with improved alkaline stability compared with wild-type FpL_C3 (having no amino acid substitution) (SEQ ID NO: 1) were selected therefrom.
(6) The selected transformants were cultured, and expression vectors were prepared using a QIAprep Spin Miniprep kit (manufactured by QIAGEN N.V.).
(7) For the sequence of the immunoglobulin-binding protein-encoding polynucleotide region inserted in the resulting expression vectors, the positions of amino acid substitutions were identified by analyzing the nucleotide sequence by the method described in Example 1 (5).
(8) The immunoglobulin-binding protein-expressing transformants selected in (5) were each inoculated into 2 mL of 2× YT liquid medium containing 50 µg/mL kanamycin, and preculture was performed by aerobic shake culture overnight at 37°C.
(9) Into 20 mL of 2× YT liquid medium supplemented with 50 µg/mL kanamycin, 200 µL of the preculture broth was inoculated. Then aerobic shake culture was performed at 37°C.
(10) 2 hours after the start of culturing, the culture temperature was changed to 20°C, and IPTG was added to a final concentration of 0.01 mM. Then aerobic shake culture was performed overnight at 20°C.
(11) After completion of culturing, the bacterial cells were harvested by centrifugation, and protein extracts were prepared using the BugBuster Protein Extraction Reagent (manufactured by Merck KGaA).
(12) The antibody-binding activity of the immunoglobulin-binding proteins in the protein extracts prepared in (11) was measured using ELISA as described in (4).
(13) The protein extracts were diluted with TBS such that the concentration of each protein was equal. The diluted extracts were divided into two equal portions, and an equal volume of 0.2 M NaOH was added to one portion (alkali treatment), while an equal volume of TBS was added to the other (no alkali treatment). After mixing, the solutions were allowed to stand at 25°C for 5 hours.
(14) After a four-fold volume of 1 M Tris buffer (pH 7.0) was added, the antibody-binding activity of the protein solution after alkali treatment (treated with NaOH at a final concentration of 0.1 M at 25°C for 5 hours) and the protein solution without alkali treatment was measured by ELISA as described in (4). The remaining activity was calculated by dividing the antibody-binding activity of the immunoglobulin-binding protein after alkali treatment by the antibody-binding activity of the immunoglobulin-binding protein without alkali treatment. In this manner, alkaline stability was evaluated.

The results are presented in Table 2. It can be concluded that immunoglobulin-binding proteins having, in the amino acid sequence set forth in SEQ ID NO: 1, one or more amino acid substitutions selected from at least P3S (this notation denotes that the amino acid residue corresponding to proline at position 3 of SEQ ID NO: 1 has been substituted with serine; the same applies hereinafter), E5V, E8G, I17F, E27G, A41T, N44S, E49V, N50S, N50Y, N50K, N50D, E52V, E52G, T54I, N62Y and N65Y exhibited improved alkaline stability compared with wild-type FpL_C3 (SEQ ID NO: 1).

In particular, with an immunoglobulin-binding protein (SEQ ID NO: 11; designated as FpL_C3 2a) having the amino acid substitutions N50Y and E52G, a significant improvement in alkaline stability was observed (wild-type FpL_C3, 40%; FpL_C3 2a, 94%). Given that the improvement in alkaline stability in the case of an immunoglobulin-binding protein having the amino acid substitution E52G alone (SEQ ID NO: 16) was not significant (wild-type FpL_C3, 40%; SEQ ID NO: 16: 48%), the inventors presume that N50Y is the amino acid substitution responsible for the significant improvement in alkaline stability.

### [Table 2]

**Table 2**

| Amino acid substitutions | SEQ ID NO | Remaining activity [%] |
|---|---|---|
| FpL_C3 (wild-type) | 1 | 40 |
| N62Y | 5 | 62 |
| I17F, N50S | 6 | 70 |
| K22R, K29R, N44S | 7 | 74 |
| E8G, E52V, N62Y | 8 | 61 |
| E49V | 9 | 60 |
| K22R | 10 | 62 |
| N50Y, E52G | 11 | 94 |
| E27G, A41T, N50K, N65Y | 12 | 72 |
| N50S | 13 | 70 |
| E5V | 14 | 43 |
| P3S, N50D, T54I | 15 | 70 |
| E52G | 16 | 48 |
| K38R | 17 | 52 |

| | | |
|---|---|---|
| *** Alkali treatment: 25°C, 0.1 M NaOH (final concentration), 5 h** | | |

### EXAMPLE 4 Preparation of an FpL_C3 Amino Acid Substitution-Integrated Mutant (Part 1)

(1) An immunoglobulin-binding protein in which all lysine residues of FpL_C3 (SEQ ID NO: 1) (specifically, the lysine residues at positions 7, 13, 22, 29, 38, 48 and 67 of SEQ ID NO: 1) were substituted with arginine residues was prepared (SEQ ID NO: 18; hereinafter also denoted as "FpL_C3KR") .
(2) The amino acid substitutions found to be responsible for improved alkaline stability of immunoglobulin-binding proteins in Example 2 were integrated into FpL_C3KR (SEQ ID NO: 18) in an attempt to further improve stability. Specifically, a protein (designated as FpL_C3KR 3a) in which the amino acid substitutions N50Y, E52G and N62Y were introduced into FpL_C3KR was prepared.

### EXAMPLE 5 Preparation of an FpL_C3 Amino Acid Substitution-Integrated Mutant (Part 2)

(1) An immunoglobulin-binding protein FpL_C3KX (SEQ ID NO: 20) in which some of the arginine residues that had replaced lysine residues in FpL_C3KR (SEQ ID NO: 18) were substituted with other amino acid residues (specifically, a protein having the amino acid substitutions K(R)7A (this notation denotes that the amino acid residue corresponding to lysine at position 7 of SEQ ID NO: 1 was once substituted with arginine and then substituted with alanine; the same applies hereinafter), K(R)22Q and K(R)29I) was prepared.
(2) The amino acid substitutions found to be responsible for improved alkaline stability of immunoglobulin-binding proteins in Example 2 were integrated into FpL_C3KX (SEQ ID NO: 20). Specifically, (r) a protein (designated as FpL_C3KX 3a) in which the amino acid substitutions N50Y, E52G and N62Y were introduced into FpL_C3KX was prepared.

### EXAMPLE 6 Evaluation of Alkaline Stability of the FpL_C3 Amino Acid Substitution-Integrated Mutants

(1) Transformants each expressing one of wild-type FpL_C3 (SEQ ID NO: 1), prepared in Example 1, mutant-type (amino acid substitution) immunoglobulin-binding protein FpL_C3KR (SEQ ID NO: 18) or FpL_C3KR 3a (SEQ ID NO: 19), prepared in Example 4, or mutant-type immunoglobulin-binding protein FpL_C3KX (SEQ ID NO: 20) or FpL_C3KX 3a (SEQ ID NO: 21), prepared in Example 5, were cultured by the same method as in Example 3 (8) to (10), and protein extracts were prepared by the method described in Example 3 (11).
(2) The antibody-binding activity of the immunoglobulin-binding proteins in the protein extracts prepared in (1) was measured using ELISA as described in Example 3 (4).
(3) Alkali treatment was performed by the same method as in Example 3 (13) except that the concentration of NaOH used for the treatment was changed to 0.4 M (final concentration, 0.2 M), and the treatment duration was changed to 15 hours. Then the remaining activity was calculated by the same method as in Example 3 (14). In this manner, alkaline stability was evaluated.

The results are presented in Table 3. It can be concluded that immunoglobulin-binding proteins having all of the amino acid substitutions N50Y, E52G and N62Y (FpL_C3KR 3a and FpL_C3KX 3a) exhibited particularly improved alkaline stability compared with immunoglobulin-binding protein having no such amino acid substitutions (FpL_C3KR, 54%; FpL_C3KR 3a, 86%) (FpL_C3KX, 10%; FpL_C3KX 3a, 69%).

### [Table 3]

**Table 3**

| Name | Amino acid substitutions | | | | | | | | | | SEQ ID NO | Remaining activity [%] |
|---|---|---|---|---|---|---|---|---|---|---|---|---|
| | K7 | K13 | K22 | K29 | K38 | K48 | N50 | E52 | N62 | K67 | | |
| FpL_C3 | - | - | - | - | - | - | - | - | - | - | 1 | 8 |
| FpL_C3KR | R | R | R | R | R | R | - | - | - | R | 18 | 54 |
| FpL_C3KR 3a | R | R | R | R | R | R | Y | G | Y | R | 19 | 86 |
| FpL_C3KX | A | R | Q | I | R | R | - | - | - | R | 20 | 10 |
| FpL_C3KX 3a | A | R | Q | I | R | R | Y | G | Y | R | 21 | 69 |

| | | | | | | | | | | | | |
|---|---|---|---|---|---|---|---|---|---|---|---|---|
| * **Alkali treatment: 25°C, 0.2 M NaOH (final concentration), 15 h** | | | | | | | | | | | | |

### EXAMPLE 7 Screening of FpL_C3KX 3a Amino Acid Substitution Mutants

(1) A library was constructed by inserting a polynucleotide encoding FpL_C3KX 3a (SEQ ID NO: 21) into the plasmid pET-26b and randomly introducing mutations by error-prone PCR into the polynucleotide portion of the resulting vector pET-FpL_C3KX, capable of expressing FpL_C3KX 3a, using the same method as in Example 2. The average mutation introduction rate was 1.7%.
(2) A transformant capable of expressing FpL_C3KX 3a (SEQ ID NO: 21), obtained by transforming the Escherichia coli strain BL21(DE3) with pET-FpL_C3KX 3a, and the random mutant library (transformants) prepared in (1) were cultured by the same method as in Example 3 (1) and (2).
(3) After culturing, the culture supernatant was obtained through a centrifugation operation and mixed with an equal volume of 1.0 M NaOH, and the resulting mixture was subjected to alkali treatment at 42°C for 30 minutes.
(4) The antibody-binding activity of the immunoglobulin-binding protein after alkali treatment was measured by ELISA as described in Example 3 (4), and the remaining activity was calculated by dividing the antibody-binding activity of the immunoglobulin-binding protein after alkali treatment by the antibody-binding activity of the immunoglobulin-binding protein without alkali treatment.
(5) Approximately 1400 strains of transformants were evaluated, and transformants expressing immunoglobulin-binding proteins with improved alkaline stability compared with FpL_C3KX 3a (SEQ ID NO: 21) were selected therefrom.
(6) The selected transformants or the transformant expressing FpL_C3KX 3a (SEQ ID NO: 21) was cultured, and expression vectors were prepared using a QIAprep Spin Miniprep kit (manufactured by QIAGEN N.V.).
(7) For the sequence of the immunoglobulin-binding protein-encoding polynucleotide region inserted in the resulting expression vectors, the positions of amino acid mutations were identified by analyzing the nucleotide sequence by the method described in Example 1 (5).
(8) The immunoglobulin-binding protein-expressing transformants selected in (1) were cultured by the same method as in Example 3 (8) to (10), and protein extracts were prepared by the same method as in Example 3 (11).
(9) The antibody-binding activity of the immunoglobulin-binding proteins in the protein extracts prepared in (8) was measured using ELISA as described in Example 3 (4).
(10) Alkali treatment was performed by the same method as in Example 3 (13) except that the concentration of NaOH used for the treatment was changed to 0.4 M (final concentration, 0.2 M), and the treatment duration was changed to 15 hours. Then the remaining activity was calculated by the same method as in Example 3 (14). In this manner, alkaline stability was evaluated.

The results are presented in Table 4. It can be concluded that immunoglobulin-binding proteins having, in the amino acid sequence set forth in SEQ ID NO: 21, one or more amino acid substitutions selected from at least E4K, E27R and T36A exhibited improved alkaline stability even compared with FpL_C3KX 3a (SEQ ID NO: 21), which had higher alkaline stability than wild-type FpL_C3 (SEQ ID NO: 1).

### [Table 4]

**Table 4**

| Amino acid substitutions (vs. FpL_C3KX 3a) | SEQ ID NO | Remaining activity [%] |
|---|---|---|
| No substitutions (FpL_C3KX 3a) | 21 | 65 |
| E4K | 22 | 69 |
| K(Q)22T | 23 | 82 |
| E27R | 24 | 72 |
| T36A | 25 | 68 |
| E49D | 26 | 64 |

| | | |
|---|---|---|
| *** Alkali treatment: 25°C, 0.2 M NaOH (final concentration), 15 h** | | |

### EXAMPLE 8 Evaluation of Affinity for Immunoglobulin κ Light Chains of Immunoglobulin-Binding Proteins (Part 1)

The binding activity to immunoglobulin κ light chains of mutant-type immunoglobulin-binding proteins was measured by ELISA as described below, and the influence of amino acid substitutions on binding activity to human immunoglobulin κ light chains was evaluated.

(1) Transformants each expressing one of FpL_C3KR (SEQ ID NO: 18), prepared in Example 4, FpL_C3KX 3a (SEQ ID NO: 21), prepared in Example 5, or an immunoglobulin-binding protein in which the amino acid substitution E49D was introduced into FpL_C3KX 3a, obtained in Example 7 (SEQ ID NO: 26; hereinafter also referred to as "FpL_C3KX 4h"), were cultured by the same method as in Example 3 (8) to (10), and protein extracts were prepared by the same method as in Example 3 (11). The extracts were centrifuged, and the resulting supernatants were clarified by filtration.
(2) The supernatants clarified in (1) were applied to a column packed with Ni-NTA Sepharose 6 Fast Flow (manufactured by Cytiva) pre-equilibrated with 0.05 M Tris buffer (pH 7.5) containing 0.5 M NaCl and 0.02 M imidazole (hereinafter referred to as washing buffer B). Then the column was washed with a volume of washing buffer B corresponding to ten times that of the support packed in the column. Subsequently, 0.05 M Tris buffer (pH 7.5) containing 0.5 M NaCl and 0.5 M imidazole was passed, and the fraction corresponding to the immunoglobulin-binding protein was collected.
(3) Each mutant-type immunoglobulin-binding protein contained in the collected fraction was quantified by measuring the absorbance of the fraction using a spectrophotometer. SDS-PAGE (SDS-polyacrylamide electrophoresis), furthermore, was also performed, confirming that each mutant-type immunoglobulin-binding protein was purified with high purity.
(4) The binding activity to human immunoglobulin κ light chains of the mutant-type immunoglobulin-binding proteins was measured by ELISA as described below, and the influence of amino acid substitutions on binding activity to human immunoglobulin κ light chains was evaluated.
(4-1) A monoclonal antibody solution prepared to 10 µg/mL using TBS was dispensed into wells of a 96-well microplate and immobilized (4°C, 16 hours). The polyclonal antibody used was one of the following:
   Herceptin, which included human κ light chain subgroup 1 (Vκ1) (manufactured by Chugai Pharmaceutical Co., Ltd.);
   the Human PDGFR alpha antibody, which included human κ light chain subgroup 3 (Vκ3) (manufactured by R&D Systems, Inc.);
   the Human CTLA4 antibody, which included human κ light chain subgroup 3 (Vκ3) (manufactured by R&D Systems, Inc.); and
   the Human PCSK9 antibody, which included human κ light chain subgroup 4 (Vκ4) (manufactured by R&D Systems, Inc.). After immobilization, blocking was performed using skim milk (manufactured by Becton, Dickinson and Company) prepared to 2% (w/v) using TBS.
(4-2) The wells of the 96-well microplate were washed with washing buffer A (0.05 M Tris buffer (pH 7.5) containing 0.15 M NaCl and 0.05% (w/v) Tween 20 (trade name)). Then a solution containing the immunoglobulin-binding protein under evaluation for antibody-binding activity, diluted to 50 µg/mL, was added, and the immunoglobulin-binding protein and the immobilized polyclonal antibody were allowed to react (30°C, 1 hour).
(4-3) After completion of the reaction, the wells were washed with washing buffer A, and 100 µL/well of Anti-6-His Antibody (manufactured by BETHYL LABORATORIES, Inc.), diluted to 100 ng/mL, was added and allowed to react (30°C, 1 hour).
(4-4) After completion of the reaction, the wells were washed with washing buffer A, and 50 µL/well of TMB Peroxidase Substrate (manufactured by KPL, Inc.) was added. Then the reaction was terminated by adding 50 µL/well of 1 M phosphoric acid, and the absorbance at 450 nm was measured using a microplate reader (manufactured by Tecan Trading AG) .
(4-5) The difference in absorbance between the wells in which the polyclonal antibody was immobilized and the wells in which the antibody was not immobilized was calculated, and the affinity between the monoclonal antibody and the mutant-type immunoglobulin-binding protein was evaluated based on the calculated absorbance. Differences in absorbance between the monoclonal antibodies indicate affinities for different human κ light chains. When variations in this value are large among the polyclonal antibodies, it indicates reduced affinity for particular human κ light chain subgroup(s).

The results are presented in Table 5. Given that FpL_C3KX_4h (SEQ ID NO: 26) exhibited small variations among the monoclonal antibodies, it can be concluded that this protein can bind to human immunoglobulin κ light chains regardless of their subgroup. By contrast, FpL_C3KR (SEQ ID NO: 18) and FpL_C3KX 3a (SEQ ID NO: 21) exhibited reduced binding potential, particularly for human Vκ3. Based on these results, it can be understood that binding potential for Vκ3 can be imparted by introducing the amino acid substitution E49D.

### [Table 5]

**Table 5**

| Name | SEQ ID NO | Affinity for monoclonal antibodies (absorbance at 450 nm) | | | |
|---|---|---|---|---|---|
| | | Herceptin (Vκ1) | PDGF R alpha (Vk3) | Human CTLA4 (VK3) | Human PCSK9 (Vκ4) |
| FpL_C3KR | 18 | 0.92 | 0.21 | 0.32 | 0.77 |
| FpL_C3KX 3a | 21 | 0.87 | 0.12 | 0.21 | 0.76 |
| FpL_C3KX 4h | 26 | 0.69 | 0.56 | 0.63 | 0.62 |

### EXAMPLE 9 Screening of FpL_C3KX 4h Amino Acid Substitution Mutants

(1) A library was constructed by randomly introducing mutations by error-prone PCR into the immunoglobulin-binding protein-encoding polynucleotide portion (SEQ ID NO: 27) of a vector pET-FpL_C3KX 4h expressing FpL_C3KX 4h (SEQ ID NO: 26), obtained in Example 7, using the same method as in Example 2. The average mutation introduction rate was 1.7%.
(2) The random mutant library (transformants) prepared in (1) was cultured by the same method as in Example 3 (1) and (2). Then the culture supernatant was obtained through a centrifugation operation and subjected to the alkali treatment described in Example 19 (2).
(3) The antibody-binding activity of the immunoglobulin-binding protein after alkali treatment was measured by ELISA as described in Example 3 (4), and the remaining activity was calculated by dividing the antibody-binding activity of the immunoglobulin-binding protein after alkali treatment by the antibody-binding activity of the immunoglobulin-binding protein without alkali treatment.
(4) Approximately 900 strains of transformants were evaluated, and transformants expressing immunoglobulin-binding proteins with improved alkaline stability compared with the immunoglobulin-binding protein that exhibited improved alkaline stability (FpL_C3KX 4h) were selected therefrom.
(5) The selected transformants were cultured, and expression vectors were prepared using a QIAprep Spin Miniprep kit (manufactured by QIAGEN N.V.).
(6) For the sequence of the immunoglobulin-binding protein-encoding polynucleotide region inserted in the resulting expression vectors, the positions of amino acid mutations were identified by analyzing the nucleotide sequence by the method described in Example 1 (5).
(7) The immunoglobulin-binding protein-expressing transformants selected in (6) or a transformant expressing FpL_C3KX 4h (SEQ ID NO: 26), obtained in Example 7, was cultured by the same method as in Example 3 (8) to (10), and protein extracts were prepared by the same method as in Example 3 (11). The extracts were centrifuged, and the resulting supernatants were clarified by filtration.
(8) The supernatants clarified in (7) were purified by the method described in Example 8 (2), and the fraction corresponding to the immunoglobulin-binding protein was collected.
(9) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(10) Alkali treatment was performed by the same method as in Example 3 (13) except that the concentration of NaOH used for the treatment was changed to 1.0 M (final concentration, 0.5 M), and the treatment duration was changed to 15 hours. Then the remaining activity was calculated by the same method as in Example 3 (14). In this manner, alkaline stability was evaluated.

The results are presented in Table 6. It can be concluded that immunoglobulin-binding proteins having, in the amino acid sequence set forth in SEQ ID NO: 26, one or more amino acid substitutions selected from at least E9V, Y53F, T54M and A69T exhibited improved alkaline stability even compared with FpL_C3KX 4h (SEQ ID NO: 26), which had higher alkaline stability than wild-type FpL_C3 (SEQ ID NO: 1).

It should be noted that when the purification yield after culturing in a 20-mL medium was compared, it was found that introducing the amino acid substitution K(Q)22R significantly improves the purification yield of the protein. Hereinafter, an immunoglobulin-binding protein in which the amino acid substitution K(Q)22R has been introduced into FpL_C3KX 4h is designated as FpL_C3KX 4i (SEQ ID NO: 29).

### [Table 6]

**Table 6**

| Amino acid substitutions (vs. FpL_C3KX 4h) | SEQ ID NO | Remaining activity [%] | Purified protein yield [mg/20 mL (medium)] |
|---|---|---|---|
| No substitutions (FpL_C3KX 4h) | 26 | 45 | 1.57 |
| E9V | 28 | 59 | 0.43 |
| K(Q)22R | 29 | 59 | 4.52 |
| Y53F | 30 | 55 | 1.18 |
| T54M | 31 | 59 | 1.09 |
| A69T | 32 | 47 | 0.58 |

| | | | |
|---|---|---|---|
| * **Alkali treatment: 25°C, 0.5 M NaOH (final concentration), 15 h** | | | |

### EXAMPLE 10 Preparation of an FpL_C3KX 4i Amino Acid Substitution-Integrated Mutant

The amino acid substitutions found to be responsible for improved alkaline stability of immunoglobulin-binding proteins in Example 9 were integrated into FpL_C3KX 4i (SEQ ID NO: 29) in an attempt to further improve stability. Specifically, a protein (SEQ ID NO: 33; designated as FpL_C3KX 5e) in which the amino acid substitution Y53F was introduced into FpL_C3KX 4i was designed and prepared.

### EXAMPLE 11 Evaluation of Alkaline Stability of FpL_C3KX 4h Amino Acid Substitution Mutants

(1) Transformants each expressing one of FpL_C3KX 4h (SEQ ID NO: 26), obtained in Example 7, FpL_C3KX 4i (SEQ ID NO: 29), obtained in Example 9, or FpL_C3KX 5e (SEQ ID NO: 33), prepared in Example 10, were cultured by the same method as in Example 3 (8) to (10), and protein extracts were prepared by the same method as in Example 3 (11). The extracts were centrifuged, and the resulting supernatants were clarified by filtration.
(2) The supernatants clarified in (1) were purified by the method described in Example 8 (2), and the fraction corresponding to the immunoglobulin-binding protein was collected.
(3) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(4) Alkali treatment was performed by the same method as in Example 3 (13) except that the concentration of NaOH used for the treatment was changed to 1.0 M (final concentration, 0.5 M), the treatment temperature was changed to 55°C, and the treatment duration was changed to 15 hours. Then the remaining activity was calculated by the same method as in Example 3 (14). In this manner, alkaline stability was evaluated.

The results are presented in Table 7. It can be concluded that FpL_C3KX 5e (SEQ ID NO: 33), prepared in Example 10, exhibited improved alkaline stability even compared with FpL_C3KX 4h (SEQ ID NO: 26) and FpL_C3KX 4i (SEQ ID NO: 29), which had higher alkaline stability than wild-type FpL_C3 (SEQ ID NO: 1) (FpL_C3KX 4h, 34%; FpL_C3KX 4i, 48%; FpL_C3KX 5e, 73%).

### [Table 7]

**Table 7**

| Name | Amino acid substitutions | | SEQ ID NO | Remaining activity [%] |
|---|---|---|---|---|
| | K22 | Y53 | | |
| FpL_C3KX 4h | Q | - | 26 | 34 |
| FpL_C3KX 4i | R | - | 29 | 48 |
| FpL_C3KX 5e | R | F | 33 | 73 |

| | | | | |
|---|---|---|---|---|
| * **Alkali treatment: 55°C, 0.5 M NaOH (final concentration), 15 min** | | | | |

### EXAMPLE 12 Screening of FpL_C3KX 5e Amino Acid Substitution Mutants

(1) A library was constructed by randomly introducing mutations by error-prone PCR into the immunoglobulin-binding protein-encoding polynucleotide portion (SEQ ID NO: 34) of a vector pET-FpL_C3KX 5e expressing FpL_C3KX 5e (SEQ ID NO: 33), obtained in Example 10, using the same method as in Example 2. The average mutation introduction rate was 1.9%.
(2) The random mutant library (transformants) prepared in (1) was cultured by the same method as in Example 3 (1) and (2). Then the culture supernatant was obtained through a centrifugation operation and subjected to the alkali treatment described in Example 19 (2).
(3) The antibody-binding activity of the immunoglobulin-binding protein after alkali treatment was measured by ELISA as described in Example 3 (4), and the remaining activity was calculated by dividing the antibody-binding activity of the immunoglobulin-binding protein after alkali treatment by the antibody-binding activity of the immunoglobulin-binding protein without alkali treatment.
(4) Approximately 900 strains of transformants were evaluated, and transformants expressing immunoglobulin-binding proteins with improved alkaline stability compared with the immunoglobulin-binding protein that exhibited improved alkaline stability (FpL_C3KX 5e) were selected therefrom.
(5) The selected transformants were cultured, and expression vectors were prepared using a QIAprep Spin Miniprep kit (manufactured by QIAGEN N.V.).
(6) For the sequence of the immunoglobulin-binding protein-encoding polynucleotide region inserted in the resulting expression vectors, the positions of amino acid mutations were identified by analyzing the nucleotide sequence by the method described in Example 1 (5).
(7) The immunoglobulin-binding protein-expressing transformants selected in (4) or a transformant expressing FpL_C3KX 5e (SEQ ID NO: 33), prepared in Example 10, was cultured by the same method as in Example 3 (8) to (10), and protein extracts were prepared by the same method as in Example 3 (11). The extracts were centrifuged, and the resulting supernatants were clarified by filtration.
(8) The supernatants clarified in (7) were purified by the method described in Example 8 (2), and the fraction corresponding to the immunoglobulin-binding protein was collected.
(9) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(10) Alkali treatment was performed by the same method as in Example 3 (13) except that the concentration of NaOH used for the treatment was changed to 2.0 M (final concentration, 1.0 M), and the treatment duration was changed to 17 hours. Then the remaining activity was calculated by the same method as in Example 3 (14). In this manner, alkaline stability was evaluated.

The results are presented in Table 8. It can be concluded that immunoglobulin-binding proteins having, in the amino acid sequence set forth in SEQ ID NO: 33, one or more amino acid substitutions selected from at least E1V, E4G, G21R and E(G)52D exhibited improved alkaline stability even compared with FpL_C3KX 5e (SEQ ID NO: 33), which had higher alkaline stability than wild-type FpL_C3 (SEQ ID NO: 1).

### [Table 8]

**Table 8**

| Amino acid substitutions (vs. FpL_C3KX 5e) | SEQ ID NO | Remaining activity [%] |
|---|---|---|
| No substitutions (FpL_C3KX 5e) | 33 | 41 |
| E1V | 35 | 44 |
| E4G | 36 | 45 |
| K(A)7S | 37 | 43 |
| G21R | 38 | 42 |
| E(G)52D | 39 | 49 |

| | | |
|---|---|---|
| * **Alkali treatment: 25°C, 1.0 M NaOH (final concentration), 17 h** | | |

### EXAMPLE 13 Evaluation of Affinity for Immunoglobulin κ Light Chains of Immunoglobulin-Binding Proteins (Part 2)

The influence of the introduction of the amino acid substitution P6S on binding activity to human immunoglobulin κ light chains was evaluated. Specifically, an immunoglobulin-binding protein (designated as FpL_C3KX 5e_P6S; SEQ ID NO: 40) in which the amino acid substitution P6S was introduced into FpL_C3KX 5e (SEQ ID NO: 33) was prepared and evaluated.

(1) Transformants each expressing one of FpL_C3KX_5e_P6S (SEQ ID NO: 40) or FpL_C3KX_5e (SEQ ID NO: 33) were cultured by the same method as in Example 3 (8) to (10), and protein extracts were prepared by the same method as in Example 3 (11). The extracts were centrifuged, and the resulting supernatants were clarified by filtration.
(2) The supernatants clarified in (1) were purified by the method described in Example 8 (2), and the fraction corresponding to the immunoglobulin-binding protein was collected.
(3) Each mutant-type immunoglobulin-binding protein contained in the collected fraction was quantified by measuring the absorbance of the fraction using a spectrophotometer. SDS-PAGE, furthermore, was also performed, confirming that each mutant-type immunoglobulin-binding protein was purified with high purity.
(4) The binding activity to human immunoglobulin κ light chains of the immunoglobulin-binding proteins was measured by the same method as in Example 8 (4), and the influence of the amino acid substitution on binding activity to human immunoglobulin κ light chains was evaluated. It should be noted that the monoclonal antibody used for the evaluation of binding activity was one of the following:
   Herceptin, which included human κ light chain subgroup 1 (Vκ1) (manufactured by Chugai Pharmaceutical Co., Ltd.); and
   the Human CTLA4 antibody, which included human κ light chain subgroup 3 (Vκ3) (manufactured by R&D Systems, Inc.).

### REFERENCE EXAMPLE 1

The influence of the introduction of the amino acid substitution K38E on binding activity to human immunoglobulin κ light chains was evaluated. Specifically, an immunoglobulin-binding protein (SEQ ID NO: 41; designated as FpL_C3KR_K38E) in which the amino acid substitution K(R)38E was introduced into FpL_C3KR (SEQ ID NO: 18) was prepared and evaluated.

(1) Transformants each expressing one of FpL_C3KR_K38E (SEQ ID NO: 41) or FpL_C3KR (SEQ ID NO: 18), prepared in Example 4, were cultured by the same method as in Example 3 (8) to (10), and protein extracts were prepared by the same method as in Example 3 (11). The extracts were centrifuged, and the resulting supernatants were clarified by filtration.
(2) The supernatants clarified in (1) were purified by the method described in Example 8 (2), and the fraction corresponding to the immunoglobulin-binding protein was collected.
(3) Each mutant-type immunoglobulin-binding protein contained in the collected fraction was quantified by measuring the absorbance of the fraction using a spectrophotometer. SDS-PAGE, furthermore, was also performed, confirming that each mutant-type immunoglobulin-binding protein was purified with high purity.
(4) The binding activity to human immunoglobulin κ light chains of the immunoglobulin-binding proteins was measured by the same method as in Example 13 (4), and the influence of the amino acid substitution on binding activity to human immunoglobulin κ light chains was evaluated.

The results for Example 13 and Reference Example 1 are presented together in Table 9. It can be understood that binding potential for Vκ3 can be imparted by introducing the amino acid substitution P6S (Example 13) or K(R)38E (Reference Example 1).

### [Table 9]

**Table 9**

| Name | | SEQ ID NO | Affinity for monoclonal antibodies (absorbance at 450 nm) | |
|---|---|---|---|---|
| | | | Herceptin (Vκ1) | Human CTLA4 (Vκ3) |
| Example 13 | | | | |
| | FpL_C3KX 5e | 33 | 0.96 | 0.06 |
| | FpL_C3KX 5e_P6S | 40 | 0.98 | 0.78 |

| Reference Example 1 | | | | |
|---|---|---|---|---|
| | FpL_C3KR | 18 | 0.86 | 0.07 |
| | FpL_C3KR_K38E | 41 | 1.02 | 0.98 |

### EXAMPLE 14 Preparation of an FpL_C3KX 5e Amino Acid Substitution-Integrated Mutant

The above amino acid substitutions that impart binding potential for Vκ3 and the amino acid substitutions found to be responsible for improved alkaline stability of immunoglobulin-binding proteins in Example 27 were integrated into FpL_C3KX 5e. Specifically, a protein (SEQ ID NO: 42; designated as FpL_C3KX 7d) in which the amino acid substitutions E4G, P6S, K(R)38E and E(G)52D were introduced into FpL_C3KX 5e was designed and prepared.

### EXAMPLE 15 Evaluation of Alkaline Stability of the FpL_C3KX 5e Amino Acid Substitution-Integrated Mutant

(1) Transformants each expressing one of FpL_C3KX 5e (SEQ ID NO: 33), prepared in Example 10, or FpL_C3KX 7d (SEQ ID NO: 42), prepared in Example 14, were cultured by the same method as in Example 3 (8) to (10), and protein extracts were prepared by the same method as in Example 3 (11). The extracts were centrifuged, and the resulting supernatants were clarified by filtration.
(2) The supernatants clarified in (1) were purified by the method described in Example 8 (2), and the fraction corresponding to the immunoglobulin-binding protein was collected.
(3) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(4) Alkali treatment was performed by the same method as in Example 3 (13) except that the concentration of NaOH used for the treatment was changed to 1.0 M (final concentration, 0.5 M), and the treatment duration was changed to 15 hours. Then the remaining activity was calculated by the same method as in Example 3 (14). In this manner, alkaline stability was evaluated.

The results are presented in Table 10. It can be concluded that FpL_C3KX 7d (SEQ ID NO: 42), evaluated in this example, exhibited improved alkaline stability even compared with FpL_C3KX 5e (SEQ ID NO: 33), which had higher alkaline stability than wild-type FpL_C3 (SEQ ID NO: 1).

### [Table 10]

**Table 10**

| Name | Amino acid substitutions (vs. FpL_C3KX 5e) | SEQ ID NO | Remaining activity [%] |
|---|---|---|---|
| FpL_C3KX 5e | - | 33 | 54 |
| FpL_C3KX 7d | E4G, P6S, K(R)38E, E(G)52D | 42 | 59 |

| | | | |
|---|---|---|---|
| * **Alkali treatment: 25°C, 0.5 M NaOH (final concentration), 15 h** | | | |

### EXAMPLE 16 Screening of FpL_C3KX 7d Amino Acid Substitution Mutants (Part 1)

(1) A library was constructed by randomly introducing mutations by error-prone PCR into the immunoglobulin-binding protein-encoding polynucleotide portion (SEQ ID NO: 43) of a vector pET-FpL_C3KX 7d expressing FpL_C3KX 7d (SEQ ID NO: 42), prepared in Example 14, using the same method as in Example 2. The average mutation introduction rate was 1.9%.
(2) A transformant capable of expressing FpL_C3KX 7d and the random mutant library (transformants) prepared in (1) were cultured by the same method as in Example 3 (1) and (2).
(3) After culturing, the culture supernatant was obtained through a centrifugation operation and mixed with an equal volume of 1.0 M NaOH, and the resulting mixture was subjected to alkali treatment at 58°C for 30 minutes.
(4) The antibody-binding activity of the immunoglobulin-binding protein after alkali treatment was measured by ELISA as described in Example 3 (4), and the remaining activity was calculated by dividing the antibody-binding activity of the immunoglobulin-binding protein after alkali treatment by the antibody-binding activity of the immunoglobulin-binding protein without alkali treatment.
(5) Approximately 1000 strains of transformants were evaluated, and transformants expressing immunoglobulin-binding proteins with improved alkaline stability compared with FpL_C3KX 7d (SEQ ID NO: 42) were selected therefrom.
(6) The selected transformants were cultured, and expression vectors were prepared using a QIAprep Spin Miniprep kit (manufactured by QIAGEN N.V.).
(7) For the sequence of the immunoglobulin-binding protein-encoding polynucleotide region inserted in the resulting expression vectors, the positions of amino acid mutations were identified by analyzing the nucleotide sequence by the method described in Example 1 (5).
(8) The immunoglobulin-binding protein-expressing transformants selected in (5) or the transformant expressing FpL_C3KX 7d (SEQ ID NO: 42) was cultured by the same method as in Example 3 (8) to (10), and protein extracts were prepared by the same method as in Example 3 (11). The extracts were centrifuged, and the resulting supernatants were clarified by filtration.
(9) The supernatants clarified in (8) were purified by the method described in Example 8 (2), and the fraction corresponding to the immunoglobulin-binding protein was collected.
(10) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(11) Alkali treatment was performed by the same method as in Example 3 (13) except that the concentration of NaOH used for the treatment was changed to 1.0 M (final concentration, 0.5 M), and the treatment duration was changed to 15 hours. Then the remaining activity was calculated by the same method as in Example 3 (14). In this manner, alkaline stability was evaluated.

The results are presented in Table 11. It can be concluded that immunoglobulin-binding proteins having, in the amino acid sequence set forth in SEQ ID NO: 42, one or more amino acid substitutions selected from at least E8G, K(I)29F, E33V and A69V exhibited improved alkaline stability even compared with FpL_C3KX 7d (SEQ ID NO: 42), which had higher alkaline stability than wild-type FpL_C3 (SEQ ID NO: 1).

### [Table 11]

**Table 11**

| Amino acid substitutions | SEQ ID NO | Remaining activity [%] |
|---|---|---|
| No substitutions (FpL_C3KX 7d) | 42 | 80 |
| A69V | 44 | 82 |
| K(I)29F | 45 | 93 |
| E8G, E33V | 46 | 85 |

| | | |
|---|---|---|
| * **Alkali treatment: 25°C, 0.5 M NaOH (final concentration), 15 h** | | |

### EXAMPLE 17 Mutagenesis of FpL_C3KX 7d and Library Construction

(1) Using a vector pET-FpL_C3KX 7d capable of expressing FpL_C3KX 7d, prepared in Example 14, as the template, a saturation substitution mutant library for the amino acid residues at positions 23, 41 and 52 was constructed. The primers were designed by arranging appropriate mixed bases at the positions where the mutations were to be introduced, such that inverse PCR was feasible around the positions for mutagenesis (SEQ ID NOs: 47 to 52, 54 to 59 and 62 to 67), according to the 22c-Trick method (Sabrina Kille et. al., ACS Synthetic Biology 2013; 2: 83-92). These primers were mixed according to the compositions specified in Table 12 and Table 13, with six primers grouped per substitution at one position. In this manner, PCR Primer mixtures (Forward/Reverse primer mixes) were prepared.

### [Table 12]

**Table 12**

| Composition | | Volume |
|---|---|---|
| Forward/reverse primer mix | | |
| | 10 µM Forward primer 1 | 24 µL |
| | 10 µM Forward primer 2 | 18 µL |
| | 10 µM Forward primer 3 | 2 µL |
| | 10 µM Reverse primer 1 | 24 µL |
| | 10 µM Reverse primer 2 | 18 µL |
| | 10 µM Reverse primer 3 | 2 µL |

### [Table 13]

**Table 13**

| | Positions of amino acid substitutions | | |
|---|---|---|---|
| | I23 | A41 | E52 |
| Forward primer 1 | 47 | 54 | 62 |
| Forward primer 2 | 48 | 55 | 63 |
| Forward primer 3 | 49 | 56 | 64 |
| Reverse primer 1 | 50 | 57 | 65 |
| Reverse primer 2 | 51 | 58 | 66 |
| Reverse primer 3 | 52 | 59 | 67 |

(2) Site-directed mutagenesis by inverse PCR was performed using pET-FpL_C3KX 7d as the template DNA. The inverse PCR was performed by preparing a reaction solution having the composition presented in Table 14, then subjecting the reaction solution to heat treatment at 98°C for 2 minutes, carrying out 30 cycles of reaction, in which a first step at 95°C for 10 seconds, a second step at 50°C for 5 seconds and a third step at 72°C for 6 minutes constituted one cycle, and finally applying heat treatment at 72°C for 7 minutes.

### [Table 14]

**Table 14**

| Composition | Volume |
|---|---|
| 1 ng/µL template DNA | 1 µL |
| Forward/reverse primer mix | 1 µL |
| PrimeSTAR HS (premix) (manufactured by Takara Bio Inc.) | 25 µL |
| H₂O | Up to 50 µL |

(3) After purification of the resulting PCR product, the Escherichia coli strain BL21(DE3) was transformed using the reaction solution and cultured on an LB plate medium containing 50 µg/mL kanamycin (37°C, 16 hours). Then the colonies that formed on the plate were defined as a site-directed mutant library.

### EXAMPLE 18 Screening of FpL_C3KX 7d Amino Acid Substitution Mutants (Part 2)

(1) The transformant capable of expressing FpL_C3KX 7d and the site-directed amino acid substitution mutant library (transformants) prepared in Example 17 were cultured by the same method as in Example 3 (1) and (2). Then the culture supernatant was obtained through a centrifugation operation and subjected to the alkali treatment described in Example 16 (3).
(2) The antibody-binding activity of the immunoglobulin-binding protein after alkali treatment was measured by ELISA as described in Example 3 (4), and the remaining activity was calculated by dividing the antibody-binding activity of the immunoglobulin-binding protein after alkali treatment by the antibody-binding activity of the immunoglobulin-binding protein without alkali treatment.
(3) Approximately 300 strains of transformants were evaluated, and transformants expressing immunoglobulin-binding proteins with improved alkaline stability compared with FpL_C3KX 7d (SEQ ID NO: 42) were selected therefrom.
(4) The selected transformants were cultured, and expression vectors were prepared using a QIAprep Spin Miniprep kit (manufactured by QIAGEN N.V.).
(5) For the sequence of the immunoglobulin-binding protein-encoding polynucleotide region inserted in the resulting expression vectors, the positions of amino acid mutations were identified by analyzing the nucleotide sequence by the method described in Example 1 (5).
(6) The immunoglobulin-binding protein-expressing transformants selected in (3) or the transformant expressing FpL_C3KX 7d (SEQ ID NO: 42), prepared in Example 14, was cultured by the same method as in Example 3 (8) to (10), and protein extracts were prepared by the same method as in Example 3 (11). The extracts were centrifuged, and the resulting supernatants were clarified by filtration.
(7) The supernatants clarified in (6) were purified by the method described in Example 8 (2), and the fraction corresponding to the immunoglobulin-binding protein was collected.
(8) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(9) Alkali treatment was performed by the same method as in Example 3 (13) except that the concentration of NaOH used for the treatment was changed to 1.0 M (final concentration, 0.5 M), and the treatment duration was changed to 15 hours. Then the remaining activity was calculated by the same method as in Example 3 (14). In this manner, alkaline stability was evaluated.

The results are presented in Table 15. It can be concluded that immunoglobulin-binding proteins having, in the amino acid sequence set forth in SEQ ID NO: 42, at least the amino acid substitutions I23R, A41I, A41Y, E(G,D)52L (this notation denotes that the amino acid residue corresponding to glutamic acid at position 7 of SEQ ID NO: 1 was once substituted with glycine, then substituted with aspartic acid and subsequently substituted with leucine; the same applies hereinafter) and E(G,D)52V exhibited improved alkaline stability even compared with FpL_C3KX 7d (SEQ ID NO: 42), which had higher alkaline stability than wild-type FpL_C3 (SEQ ID NO: 1).

### [Table 15]

**Table 15**

| Amino acid substitutions | SEQ ID NO | Remaining activity [%] |
|---|---|---|
| No substitutions (FpL_C3KX 7d) | 42 | 85 |
| I23R | 53 | 86 |
| A411 | 60 | 86 |
| A41Y | 61 | 88 |
| E(G, D)52L | 68 | 86 |
| E(G, D)52V | 69 | 86 |

| | | |
|---|---|---|
| * **Alkali treatment: 25°C, 0.5 M NaOH (final concentration), 15 h** | | |

### EXAMPLE 19 Preparation of an FpL_C3KX 7d Amino Acid Substitution-Integrated Mutant

The amino acid substitutions found to be responsible for improved alkaline stability of immunoglobulin-binding proteins in Examples 16 and 18 were integrated into FpL_C3KX 7d (SEQ ID NO: 42) in an attempt to further improve alkaline stability. Specifically, a protein (SEQ ID NO: 70; designated as FpL_C3KX 8b) in which the amino acid substitutions E(G,D)52L and A69V were introduced into FpL_C3KX 7d was designed and prepared.

### EXAMPLE 20 Evaluation of Alkaline Stability of the FpL_C3KX 7d Amino Acid Substitution-Integrated Mutant

(1) Transformants each expressing one of FpL_C3KX 7d_A69V (SEQ ID NO: 44), obtained in Example 16, or FpL_C3KX 8b (SEQ ID NO: 70), prepared in Example 18, were cultured by the same method as in Example 3 (8) to (10), and protein extracts were prepared by the same method as in Example 3 (11). The extracts were centrifuged, and the resulting supernatants were clarified by filtration.
(2) The supernatants clarified in (1) were purified by the method described in Example 8 (2), and the fraction corresponding to the immunoglobulin-binding protein was collected.
(3) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(4) Alkali treatment was performed by the same method as in Example 3 (13) except that the concentration of NaOH used for the treatment was changed to 1.0 M (final concentration, 0.5 M), and the treatment duration was changed to 15 hours. Then the remaining activity was calculated by the same method as in Example 3 (14). In this manner, alkaline stability was evaluated.

The results are presented in Table 16. It can be concluded that FpL_C3KX 8b (SEQ ID NO: 70) exhibited improved alkaline stability even compared with FpL_C3KX 7d_A69V (SEQ ID NO: 44), which had higher alkaline stability than wild-type FpL_C3 (SEQ ID NO: 1).

### [Table 16]

**Table 16**

| Name | Amino acid substitutions (vs. FpL_C3KX 7d) | SEQ ID NO | Remaining activity [%] |
|---|---|---|---|
| FpL_C3KX 7d_A69V | A69V | 44 | 75 |
| FpL_C3KX 8b | A69V, E(G, D)52L | 70 | 88 |

| | | | |
|---|---|---|---|
| * **Alkali treatment: 25°C, 0.5 M NaOH (final concentration), 15 h** | | | |

### COMPARATIVE EXAMPLE 1 Preparation of FpL_C3

(1) The recombinant Escherichia coli obtained in Example 1, capable of expressing FpL_C3, was inoculated into 20 mL of 2× YT liquid medium (1.6% (w/v) tryptone, 1% (w/v) yeast extract and 0.5% (w/v) sodium chloride) containing 50 µg/mL kanamycin and precultured (30°C, 16 hours).
(2) 10 mL of the culture broth after preculturing was transferred into 1 L of 2× YT liquid medium containing 50 µg/mL kanamycin, and the cells were cultured at 37°C for 2 hours to 3 hours. Then 200 µL of 0.5 M IPTG was added, and the cells were further shake-cultured overnight at 20°C.
(3) The culture broth after culturing was centrifuged, and the cultured bacterial cells were harvested (8 g as a wet weight). 2 g as a wet weight of the cultured bacterial cells was lysed using the BugBuster Protein Extraction Reagent (manufactured by Merck Millipore Corporation), and the supernatant was obtained by centrifugation and clarified by filtration.
(4) The supernatant clarified in (3) was applied to a column packed with Ni Sepharose 6 Fast Flow (manufactured by Cytiva) pre-equilibrated with 0.02 M Tris buffer (pH 7.5) containing 0.5 M sodium chloride and 0.02 M imidazole (hereinafter also referred to simply as "equilibrating solution"). Then the column was washed with a volume of the equilibrating solution corresponding to ten times that of the support packed in the column. Subsequently, 0.02 M Tris buffer (pH 7.5) containing 0.5 M sodium chloride and 0.5 M imidazole was passed, and the fraction corresponding to the immunoglobulin-binding protein was collected.
(5) The amount of protein contained in the collected fraction was quantified by measuring the absorbance of the fraction using a spectrophotometer. SDS-PAGE, furthermore, was also performed, confirming that the immunoglobulin-binding protein was purified with high purity.

### EXAMPLE 21 Evaluation of Affinity for Immunoglobulin κ Light Chains of Immunoglobulin-Binding Proteins (Part 3)

The binding activity to immunoglobulin κ light chains of the FpL_C3 prepared in Comparative Example 1 and FpL_C3KX 8b, prepared in Example 19, was measured by the same method as in Example 8 (4), and the influence of amino acid substitutions on binding activity to human immunoglobulin κ light chains was evaluated. It should be noted that the monoclonal antibody used for the evaluation of binding activity was one of the following:
Humira, which included human κ light chain subgroup 1 (Vκ1) (manufactured by Eisai Co., Ltd.);
the Human PDGFR alpha antibody, which included human κ light chain subgroup 3 (Vκ3) (manufactured by R&D Systems, Inc.); and
the Human PCSK9 antibody, which included human κ light chain subgroup 4 (Vκ4) (manufactured by R&D Systems, Inc.).

The results are presented in Table 17. Given that FpL_C3 (SEQ ID NO: 1) exhibited equally intense color for each monoclonal antibody, it can be concluded that this protein can bind to human immunoglobulin κ light chains regardless of their subgroup. By contrast, FpL_C3KX 8b (SEQ ID NO: 70) exhibited reduced binding potential for Vκ3 and Vκ4. From these results, it can be concluded that FpL_C3KX 8b has improved selectivity for Vκ1 compared with FpL_C3.

### [Table 17]

**Table 17**

| Name | SEQ ID NO | Affinity for monoclonal antibodies (absorbance at 450 nm) | | | Selectivity | |
|---|---|---|---|---|---|---|
| | | Humira (Vκ1 ) | PDGF R alpha (Vκ3) | Human PCSK9 (Vκ4) | Absorbance ratio (Vκ1/Vκ3) | Absorbance ratio (Vκ1/Vκ4) |
| FpL_C3 | 1 | 0.82 | 0.71 | 0.91 | 1.2 | 0.9 |
| FpL_C3KX8b | 70 | 0.65 | 0.15 | 0.17 | 4.2 | 3.7 |

### EXAMPLE 22 Screening of FpL_C3KX 8b Amino Acid Substitution Mutants

(1) Using a vector pET-FpL_C3KX 8b capable of expressing FpL_C3KX 8b, prepared in Example 19, as the template, a saturation substitution mutant library for the amino acid residues at positions 22, 38, 42 and 44 was constructed. As in Example 17 (1), the primers were designed by arranging appropriate mixed bases at the positions where the mutations were to be introduced, such that inverse PCR was feasible around the positions for mutagenesis (SEQ ID NOs: 71 to 94), according to the 22c-Trick method. These primers were mixed according to the compositions specified in Table 12 and Table 18, with six primers grouped per substitution at one position. In this manner, PCR Primer mixtures (Forward/Reverse primer mixes) were prepared.

### [Table 18]

**Table 18**

| | Positions of amino acid substitutions | | | |
|---|---|---|---|---|
| | K22 | K38 | Y42 | N44 |
| Forward primer 1 | 71 | 77 | 83 | 89 |
| Forward primer 2 | 72 | 78 | 84 | 90 |
| Forward primer 3 | 73 | 79 | 85 | 91 |
| Reverse primer 1 | 74 | 80 | 86 | 92 |
| Reverse primer 2 | 75 | 81 | 87 | 93 |
| Reverse primer 3 | 76 | 82 | 88 | 94 |

(2) Site-directed mutagenesis by inverse PCR was performed by the same method as in Example 17 (2) except that pET-FpL_C3KX 8b, prepared in Example 19, was used as the template DNA.
(3) After purification of the resulting PCR product, the Escherichia coli strain BL21(DE3) was transformed using the purified product and cultured on an LB plate medium containing 50 µg/mL kanamycin (37°C, 16 hours). Then the colonies that formed on the plate were defined as a saturation substitution mutant library.
(4) A transformant capable of expressing FpL_C3KX 8b, prepared in Example 19, and the saturation substitution mutant library (transformants) prepared in (3) were inoculated into 250 µL of 2× YT liquid medium containing 50 µg/mL kanamycin and shake-cultured overnight at 37°C using a 96-deep-well plate.
(5) After culturing, 5 µL of the culture broth was transferred into 500 µL of 2× YT liquid medium containing 50 µg/mL kanamycin, 0.3% (w/v) glycine and 0.01 mM IPTG, and the cells were further shake-cultured overnight at 20°C using a 96-deep-well plate.
(6) After culturing, the culture supernatant was obtained through a centrifugation operation and mixed with an equal volume of TBS.
(7) Antibody-binding activity to κ light chains in different Subgroups was evaluated by the same method as in Example 21, and transformants exhibiting less intense colors in the wells in which Vκ3 or Vκ4 was immobilized, compared with FpL_C3KX 8b, were selected.
(8) Approximately 200 strains of transformants were evaluated, and transformants expressing immunoglobulin-binding proteins with reduced affinity for Vκ3 or Vκ4, compared with FpL_C3KX 8b, were selected therefrom.
(9) The transformants selected in (8) were cultured, and expression vectors were prepared using a QIAprep Spin Miniprep kit (manufactured by QIAGEN N.V.).
(10) For the sequence of the immunoglobulin-binding protein-encoding polynucleotide region inserted in the resulting expression vectors, the positions of amino acid substitutions were identified by analyzing the nucleotide sequence by the method described in Example 1 (5).
(11) The immunoglobulin-binding protein-expressing transformants selected in (10) were cultured by the same method as in Example 3 (8) to (10), and the purification of protein extracts and the collection of the fraction corresponding to the immunoglobulin-binding protein were performed by the same method as in Example 8 (1) and (2).
(12) The immunoglobulin-binding protein contained in the collected fraction was quantified by measuring the absorbance of the fraction using a spectrophotometer. SDS-PAGE, furthermore, was also performed, confirming that the selected immunoglobulin-binding protein was purified with high purity.
(13) The binding activity of the immunoglobulin-binding proteins prepared in (12) to monoclonal antibodies in different subgroups was measured by the same method as in Example 21.

The results are presented in Table 19. It was confirmed that immunoglobulin-binding proteins having, in the amino acid sequence set forth in SEQ ID NO: 1, one or more amino acid substitutions selected from at least K(R)22E, K(R,E)38P, K(R,E)38D, Y42F, Y42H, Y42W and N44P can achieve better selectivity than FpL_C3KX 8b (SEQ ID NO: 70), which exhibits improved selectivity for Vκ1 compared with wild-type FpL_C3. Furthermore, it can be understood that the immunoglobulin-binding proteins having the amino acid substitution K(R,E)38D, Y42F or Y42H, in particular, exhibited markedly reduced affinity for Vκ3 and Vκ4 and, as a result, achieved higher selectivity for Vκ1.

### [Table 19]

**Table 19**

| Amino acid substitutions | SEQ ID NO | Affinity for monoclonal antibodies (absorbance at 450 nm) | | | Selectivity | |
|---|---|---|---|---|---|---|
| | | Humira (Vκ1) | PDGF R alpha (Vκ3) | Human PCSK9 (Vκ4) | Absorbance ratio (Vκ1/Vκ3) | Absorbance ratio (Vκ1/Vκ4) |
| FpL_C3 (reference) | 1 | 0.82 | 0.71 | 0.91 | 1.2 | 0.9 |
| No substitutions (FpL_C3KX 8b) | 70 | 0.65 | 0.15 | 0.17 | 4.2 | 3.7 |
| K(R)22E | 95 | 0.33 | 0.033 | 0.15 | 10 | 2.2 |
| K(R, E)38P | 96 | 0.25 | 0.023 | 0.19 | 11 | 1.3 |
| K(R, E)38D | 97 | 0.62 | 0.062 | 0.16 | 9.9 | 3.8 |
| Y42W | 98 | 0.41 | 0.047 | 0.16 | 8.6 | 2.5 |
| Y42F | 99 | 0.51 | 0.035 | 0.08 | 14 | 6.6 |
| Y42H | 100 | 0.67 | 0.04 | 0.14 | 16 | 4.7 |
| N44P | 101 | 0.47 | 0.032 | 0.21 | 14 | 2.2 |

### EXAMPLE 23 Preparation of an FpL_C3KX 8b Amino Acid Substitution-Integrated Mutant

The amino acid substitutions found to be responsible for improved selectivity for Vκ1 in Example 22 were integrated into FpL_C3KX 8b (SEQ ID NO: 70) in an attempt to further improve selectivity. Specifically, a protein (SEQ ID NO: 102; designated as FpL_C3KX 8b_K38D,Y42H) in which the amino acid substitutions K(R,E)38D and Y42H were introduced into FpL_C3KX 8b was designed.

(1) By mutagenesis with introduced inverse PCR, a polynucleotide encoding FpL_C3KX 8b_K38D,Y42H was prepared. The inverse PCR was performed using an expression vector including a polynucleotide (SEQ ID NO: 103) encoding FpL_C3KX8b_Y42H as the template DNA, by preparing a reaction solution having the composition presented in Table 20, then subjecting the reaction solution to heat treatment at 98°C for 2 minutes, carrying out 30 cycles of reaction, in which a first step at 95°C for 10 seconds, a second step at 50°C for 5 seconds and a third step at 72°C for 1 minute constituted one cycle, and finally applying heat treatment at 72°C for 1 minute.

### [Table 20]

**Table 20**

| Composition | Volume |
|---|---|
| 1 ng/µL template DNA | 1 µL |
| Forward primer (10 µM) (SEQ ID NO: 104) | 1 µL |
| Reverse primer (10 µM) (SEQ ID NO: 105) | 1 µL |
| PrimeSTAR MAX (premix) (manufactured by Takara Bio Inc.) | 25 µL |
| H₂O | Up to 50 µL |

(2) The Escherichia coli strain BL21(DE3) was transformed using the PCR product obtained in (1) and cultured on an LB (Luria-Bertani) plate medium containing 50 µg/mL kanamycin (37°C, 16 hours).
(3) The transformant (recombinant Escherichia coli) obtained in (2) was subjected to selection. Then culturing and plasmid purification were performed by the same method as in Example 1 (4), whereby a vector (designated as pET-FpL_C3KX 8b_E38D,Y42H) capable of expressing FpL_C3KX 8b_E38D,Y42H was obtained.
(4) Of the expression vector pET-FpL_C3KX 8b_E38D,Y42H obtained in (3), the polynucleotide encoding FpL_C3KX 8b_E38D,Y42H and its flanking regions were subjected to sequence analysis by the same method as in Example 1 (5).

As a result of the sequence analysis, it was confirmed that a polynucleotide (SEQ ID NO: 103) encoding FpL_C3KX8b_E38D,Y42H (SEQ ID NO: 102) had been inserted into the expression vector pET-FpL_C3KX 8b_E38D,Y42H.

(5) By the same method as in Comparative Example 1, FpL_C3KX8b_E38D,Y42H was prepared from the transformant obtained in (2).

(6) The binding activity of the immunoglobulin-binding proteins prepared in (5) to monoclonal antibodies in different subgroups was measured by the same method as in Example 21.

The results are presented in Table 21. It can be understood that FpL_C3KX8b_E38D,Y42H (SEQ ID NO: 102) exhibited lower affinity for Vκ3 and Vκ4 than FpL_C3KX 8b (SEQ ID NO: 70), which had improved selectivity for Vκ1 compared with wild-type FpL_C3, and, as a result, achieved significantly improved selectivity for Vκ1.

### [Table 21]

**Table 21**

| Name | SEQ ID NO | Affinity for monoclonal antibodies (absorbance at 450 nm) | | | Selectivity | |
|---|---|---|---|---|---|---|
| | | Humira (Vκ1) | PDGF R alpha (Vκ3) | Human PCSK9 (Vκ4) | Absorbance ratio (Vκ1/Vκ3) | Absorbance ratio (Vκ1/Vκ4) |
| FpL_C3 (reference) | 1 | 0.82 | 0.71 | 0.91 | 1.2 | 0.9 |
| No substitutions (FpL_C3KX 8b) | 70 | 0.65 | 0.15 | 0.17 | 4.2 | 3.7 |
| K(R, E)38D/Y42H | 102 | 0.52 | 0.018 | 0.062 | 29 | 8.5 |

### Industrial Applicability

The protein according to the present disclosure is characterized in that it comprises the amino acid sequence of an immunoglobulin-binding domain of Protein L derived from the genus Finegoldia, provided that in the amino acid sequence, the protein has at least one amino acid substitution that improves binding selectivity for antibodies having a particular light chain, the protein having immunoglobulin-binding activity. The immunoglobulin adsorbent according to the present disclosure, which comprises an insoluble support and the protein according to the present disclosure immobilized on the insoluble support, can be advantageous in that it enables purification and separation of bispecific antibodies, which cannot be separated with known immunoglobulin adsorbents.

## Claims

1. A protein comprising an amino acid sequence of an immunoglobulin-binding domain of Protein L derived from a bacterium of the genus Finegoldia, provided that in the amino acid sequence, the protein has one or more amino acid substitutions selected from at least (1) to (5) below, the protein having immunoglobulin-binding activity:
(1) substitution of an amino acid residue corresponding to tyrosine at position 42 of SEQ ID NO: 1 with histidine;
(2) substitution of an amino acid residue corresponding to lysine at position 22 of SEQ ID NO: 1 with glutamic acid;
(3) substitution of an amino acid residue corresponding to lysine at position 38 of SEQ ID NO: 1 with proline or aspartic acid;
(4) substitution of an amino acid residue corresponding to tyrosine at position 42 of SEQ ID NO: 1 with tryptophan or phenylalanine; and
(5) substitution of an amino acid residue corresponding to asparagine at position 44 of SEQ ID NO: 1 with proline.

2. The protein according to Claim 1, wherein the protein is a protein as described in any of (a) to (c) below:
(a) a protein including an amino acid sequence set forth in SEQ ID NO: 1, provided that in the amino acid sequence, the protein has one or more amino acid substitutions selected from at least (1) to (5) below, the protein having immunoglobulin-binding activity:
(1) substitution of an amino acid residue corresponding to tyrosine at position 42 of SEQ ID NO: 1 with histidine;
(2) substitution of an amino acid residue corresponding to lysine at position 22 of SEQ ID NO: 1 with glutamic acid;
(3) substitution of an amino acid residue corresponding to lysine at position 38 of SEQ ID NO: 1 with proline or aspartic acid;
(4) substitution of an amino acid residue corresponding to tyrosine at position 42 of SEQ ID NO: 1 with tryptophan or phenylalanine; and
(5) substitution of an amino acid residue corresponding to asparagine at position 44 of SEQ ID NO: 1 with proline;
(b) a protein including an amino acid sequence set forth in SEQ ID NO: 1, provided that in the amino acid sequence, the protein has one or more amino acid substitutions selected from at least the (1) to (5) and also includes one or several substitutions, deletions, insertions and/or additions of amino acid residues at one or several positions in addition to the (1) to (5), the protein having immunoglobulin-binding activity; and
(c) a protein including an amino acid sequence having a homology of 70% or more to an amino acid sequence set forth in SEQ ID NO: 1 or a partial sequence thereof, provided that in the amino acid sequence, the protein has one or more amino acid substitutions selected from at least the (1) to (5), the protein having immunoglobulin-binding activity.

3. The protein according to Claim 1, further comprising at least one or more amino acid substitutions selected from (6) to (11) below:
(6) substitution of an amino acid residue corresponding to asparagine at position 50 of SEQ ID NO: 1 with tyrosine;
(7) substitution of an amino acid residue corresponding to glutamic acid at position 4 of SEQ ID NO: 1 with glycine;
(8) substitution of an amino acid residue corresponding to lysine at position 7 of SEQ ID NO: 1 with alanine;
(9) substitution of an amino acid residue corresponding to glutamic acid at position 52 of SEQ ID NO: 1 with leucine;
(10) substitution of an amino acid residue corresponding to tyrosine at position 53 of SEQ ID NO: 1 with phenylalanine; and
(11) substitution of an amino acid residue corresponding to alanine at position 69 of SEQ ID NO: 1 with valine.

4. The protein according to Claim 1, further comprising at least an amino acid substitution of (6) below and one or more amino acid substitutions selected from (7) to (11) below:
(6) substitution of an amino acid residue corresponding to asparagine at position 50 of SEQ ID NO: 1 with tyrosine;
(7) substitution of an amino acid residue corresponding to glutamic acid at position 4 of SEQ ID NO: 1 with glycine;
(8) substitution of an amino acid residue corresponding to lysine at position 7 of SEQ ID NO: 1 with alanine;
(9) substitution of an amino acid residue corresponding to glutamic acid at position 52 of SEQ ID NO: 1 with leucine;
(10) substitution of an amino acid residue corresponding to tyrosine at position 53 of SEQ ID NO: 1 with phenylalanine; and
(11) substitution of an amino acid residue corresponding to alanine at position 69 of SEQ ID NO: 1 with valine.

5. The protein according to Claim 1, further comprising at least an amino acid substitution of (12) below:
(12) substitution of an amino acid residue corresponding to lysine at position 29 of SEQ ID NO: 1 with isoleucine.

6. The protein according to Claim 4, wherein the protein is a protein as described in any of (d) to (f) below:
(d) a protein including an amino acid sequence set forth in SEQ ID NO: 70, provided that in the amino acid sequence, the protein has one or more amino acid substitutions selected from at least (1) to (5) below, the protein having immunoglobulin-binding activity:
(1) substitution of an amino acid residue corresponding to tyrosine at position 42 of SEQ ID NO: 1 with histidine;
(2) substitution of an amino acid residue corresponding to lysine at position 22 of SEQ ID NO: 1 with glutamic acid;
(3) substitution of an amino acid residue corresponding to lysine at position 38 of SEQ ID NO: 1 with proline or aspartic acid;
(4) substitution of an amino acid residue corresponding to tyrosine at position 42 of SEQ ID NO: 1 with tryptophan or phenylalanine; and
(5) substitution of an amino acid residue corresponding to asparagine at position 44 of SEQ ID NO: 1 with proline;
(e) a protein including an amino acid sequence set forth in SEQ ID NO: 70, provided that in the amino acid sequence, the protein has one or more amino acid substitutions selected from at least the (1) to (5) and also includes one or several substitutions, deletions, insertions and/or additions of amino acid residues at one or several positions in addition to the (1) to (5), the protein having immunoglobulin-binding activity; and
(f) a protein including an amino acid sequence having a homology of 70% or more to an amino acid sequence set forth in SEQ ID NO: 70 or a partial sequence thereof, provided that in the amino acid sequence, the protein has one or more amino acid substitutions selected from at least the (1) to (5), the protein having immunoglobulin-binding activity.

7. A polynucleotide encoding the protein according to any one of Claims 1 to 6.

8. An expression vector comprising the polynucleotide according to Claim 7.

9. A recombinant host comprising (A) or (B) below:
(A) a polynucleotide encoding the protein according to any one of Claims 1 to 6; or
(B) an expression vector including a polynucleotide encoding the protein according to any one of Claims 1 to 6.

10. The recombinant host according to Claim 9, wherein the host is Escherichia coli.

11. A method for manufacturing a protein having immunoglobulin-binding activity, the method comprising a step of culturing a recombinant host including a polynucleotide encoding the protein according to any one of Claims 1 to 6 or a recombinant host including an expression vector including the polynucleotide to allow the protein to be expressed and a step of recovering the expressed protein.

12. The manufacturing method according to Claim 11, wherein the host is Escherichia coli.

13. An immunoglobulin adsorbent comprising an insoluble support and the protein according to any one of Claims 1 to 6 immobilized on the insoluble support.

14. A method for separating an immunoglobulin contained in a solution, the method comprising a step of applying a solution containing the immunoglobulin to a column packed with the adsorbent according to Claim 13 to allow the immunoglobulin to be adsorbed onto the adsorbent and a step of eluting the immunoglobulin adsorbed on the adsorbent.
